(19) Europäisches Patentamt
European Patent Office
Office européen des brevets

(11) EP 4 103 946 B1

(12) EUROPEAN PATENT SPECIFICATION

(45) Date of publication and mention of the grant of the patent:
**13.03.2024 Bulletin 2024/11**

(21) Application number: **21705147.3**

(22) Date of filing: **10.02.2021**

(51) International Patent Classification (IPC):
***G01N 33/68** (2006.01)*

(52) Cooperative Patent Classification (CPC):
**G01N 33/6812; G01N 33/6848**

(86) International application number:
**PCT/EP2021/053246**

(87) International publication number:
**WO 2021/160696 (19.08.2021 Gazette 2021/33)**

# (54) IN-VITRO DIAGNOSIS OF HISTAMINE INTOLERANCE SYNDROME

IN-VITRO DIAGNOSE DES HISTAMIN-INTOLERANZSYNDROMS

DIAGNOSTIC IN VITRO DU SYNDROME D’INTOLÉRANCE À L’HISTAMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.02.2020 DE 102020103325**
**08.12.2020 DE 102020132668**

(43) Date of publication of application:
**21.12.2022 Bulletin 2022/51**

(73) Proprietor: **Immundiagnostik AG**
**64625 Bensheim (DE)**

(72) Inventors:
- **ARMBRUSTER, Franz Paul**
**64270 Bobenheim-Roxheim (DE)**
- **DIESNER, Max**
**64625 Bensheim (DE)**
- **ARNOLD, Anne**
**64625 Bensheim (DE)**

(74) Representative: **Benedum, Ulrich Max**
**Nebens IP**
**Patente Marken Designs**
**Oberföhringer Strasse 172**
**81925 München (DE)**

(56) References cited:
**EP-A1- 2 020 446**
**WO-A1-2019/158718**
**US-A1- 2008 227 116**

- **ROSCOE H G ET AL: "A new histaminase assay", ANALYTICAL BIOCHEMISTRY, ACADEMIC PRESS, AMSTERDAM, NL, vol. 47, no. 2, 1 June 1972 (1972-06-01), pages 418-425, XP024816623, ISSN: 0003-2697, DOI: 10.1016/0003-2697(72)90135-2 [retrieved on 1972-06-01]**
- **LUKAS KOFLER ET AL: "Histamine 50-Skin-Prick Test: A Tool to Diagnose Histamine Intolerance", ISRN ALLERGY, vol. 115, no. 10, 22 February 2011 (2011-02-22), pages 454-5, XP055106751, ISSN: 2090-5521, DOI: 10.5402/2011/353045**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 103 946 B1

## Description

FIELD OF THE INVENTION

The present invention relates to a method and reagents for in-vitro diagnosis of histamine intolerance syndrome (WHO ICD10 - T78.1) in a sample of bodily fluid.

BACKGROUND OF THE INVENTION

Histamine intolerance, also referred to as enteral histaminosis or sensitivity to dietary histamine, is a disorder associated with an impaired ability to metabolize histamine. Histamine [2-(4-imidazolyl)-ethylamine] is formed in the body from L-histidine which is decarboxylated by the action of histidine decarboxylase (HDC, EC 4.1.1.22). This is the only endogenous source of histamine which takes part in physiological and pathophysiological processes connected with neurotransmission, gastric acid secretion and immune response. As histamine is physiologically highly active, its levels are physiologically tightly controlled at all stages: at intercellular transport, storage in secretory granules, release and by its degradation and inactivation.

The histamine intolerance syndrome is difficult to diagnose because of its multifaceted symptoms which are easily misinterpreted as symptoms of other diseases such as food allergy, intolerance to sulphides or caused by other biogenic amines such as tyramine. Histamine intolerance provokes promptly painful disorders and is a common disorder which affects about 1%-3% of the population (Sattler J et al., Food induced histaminosis as an epidemiological problem: plasma histamine elevation and hemodynamic alterations after oral histamine administration and blockade of diamine oxidase (DAO), Agents and Actions 1988, 23:361-65; Wantke F et al., The red wine maximization test: drinking histamine rich wine induces a transient increase of plasma diamine oxidase activity in healthy volunteers, Inflamm Res 1999, 48:169-70; Wantke F et al., The red wine provocation test: intolerance to histamine as a model for food intolerance, Allergy Proc 1994, 15:27-32; Wantke F et al., Daily variations of serum diamine oxidase and the influence of H1 and H2 blockers: a critical approach to routine diamine oxidase assessment, Inflamm Res 1998, 47:396-400; Jarisch R et al., Role of food allergy and food intolerance in recurrent urticaria, Curr Probl Dermatol 1999, 28:64-73, Wantke F et al., Histamine free diet: treatment of choice for histamine induced food intolerance and supporting treatment for chronical headaches, Clin Exp Allergy 1993, 23:982-85; Götz M et al., Histamin-Intoleranz und Diaminooxidasemangel, Allergologie 1996, 9:426-30; Jarisch R et al., Histamin-Intoleranz. Histamin und Seekrankheit. Stuttgart, NY, 2 ed., Georg Thieme Verl., 2004).

It is widely believed that the secreted copper-containing (AOC1) diamine oxidase (DAO - EC 1.4.3.22) is the frontline enzyme in the clearing of endogenous and exogenous histamine since blood leukocytes and tissues such as the placenta secret DAO into the circulation. Pregnant women for example have serum DAO levels 500 to 1000 times higher than non-pregnant women. On the other hand, humans have three genes encoding copper-containing amine oxidases which share large sequence identity but only one gene, the AOC1 gene, encodes said secreted human diamine oxidase (McGrath AP et al, Structure and Inhibition of Human Diamine Oxidase, Biochemistry 2009; 48:9810-22). More precisely, dietary histamine is primarily degraded by a tissue-associated DAO in the intestinal mucous membrane when it passes therethrough. Tissue-associated DAO is not merely found in the small intestine but also in liver, lung, and kidneys, and amniotic fluid contains a diamine oxidase which is immunologically different to the diamine oxidase (DAO) found in serum; see Cowley et al in US 5,284,749 A; Elmore BO et al., Human kidney diamine oxidase: heterologous expression, purification, and characterization, J Biol Inorg Chem 2002, 7:565-679; Mizuguchi et al., Purification and characterization of diamine oxidase (histaminase) from rat small intestine, J. Biochem 1994,116:631-5; Kitanaka et al., Expression of diamine oxidase (histaminase) in guinea-pig tissues, Eur J Pharmacol 2002, 437:179-85). Consequently, the serological parameters for diagnosis of histamine intolerance required a critical review which was done by a careful profiling of histamine plasma levels and DAO activities as well as clinical symptoms in patient groups: suspected histamine-intolerants, proven food-allergic patients and healthy controls (Pinzer TC et al., Circadian profiling reveals higher histamine plasma levels and lower diamine oxidase serum activities in 24% of patients with suspected histamine intolerance compared to food allergy and controls, Allergy 2018, 73:949-957). It has been found that plasma histamine allows no reliable differentiation between histamine intolerance syndrome, food hypersensitivity, food intolerance and food allergy since the pathologies are not specific but show great variability as the individual complaints by the patients. No correlations between subjective complaints and measured plasma histamine parameters have been found.

Conventional methods of as well as the diamine/histamine conversion ratio and a diamine/histamine conversion rate per unit time diagnosis of histamine intolerance syndrome are based on a measurement of histamine in plasma or serum. US 4,818,683 (Morel et al) describes an immunoassay which measures a histamine derivative. Alternative methods of measuring histamine have been described by Ellman GL, Arch Biochem Biophys 1958, 74:443-450; Riddles PW, Anal Biochem 1979, 94:75-81; Muckerheide A et al., J. Immunol 1987, 138:833; Apple RJ et al, J Immunol 1987, 140:290; Domen PJ, J Immunol 1987, 139:195; Morel and Delaage, Immunoanalysis of histamine through a novel

chemical derivatization, The Journal of Allergy and Clinical Immunology 1988, 82:646-654; Morel et al., Recognition of imidazole and histamine derivatives by monoclonal antibodies, Immunol. 1990 27:995-1000; Held, P. et al. Analysis of Histamine in Wine Samples Using the Microplate Format. BioTek Instruments Aug. 28, 2006; Kobayashi N et al, Adv Clin Chem 2001, 36:139-170; CAPLUS, STN Accession 2001: 813015; DE 695 20 383T2; RU2005114081; WO2008115044; Claret et al., Homogeneous time resolved fluorescence assay to measure histamine release, Comb Chem High Throughput Screen 2003, 6:789-94; Küfner MA et al., Determination of histamine degradation capacity in extremely small human colon samples Inflamm Res 2001, 2:596-7; Previati M et al., Determination of histamine in the whole blood of colon cancer patients, J Chromatogr, 2002, 780:331-9).

EP 1 948 819 (Sciotec Diagnostic Technologies GmbH) relates to a method for determining a DAO activity in plasma or serum. An excess of a diamine substrate, e.g. putrescine, is added to the sample and reacted by the intrinsic DAO in said sample for a defined period. The diamine being still in the sample is derivatized and determined by a competitive immunoassay. As the diamine substrate concentration stays at elevated level, the rate of DAO is proportional to its concentration in the sample and used for diagnosis. Immunoassays (ELISA) for measuring directly the amount of DAO enzyme in human serum and dried blood spots as well as of the DAO activity ($^3$H putrescine/pyrroline REA) are available from ImmundiagnostikAG, Bensheim, DE (IDK® DAO ELISA-Art. No. K8500; Art. No. K 8220 DAO REA $^3$H-putrescine ⇔ $\Delta^1$-pyrrolin; Mayer I et al., Optimierter Radioextraktionsassay zur quantitativen Bestimmung der Aktivität von Diaminooxidase (DAO) in humanem Serum und Plasma, Allergologie 2005, 28:1-8).

WO 2019/158718 (Frost Diagnostika GmbH) suggests determining an index ("percentage of the total histamine degradation") to combine the decreasing DAO rate and the decreasing diamine substrate level in a method as described in EP 1948819 B1. This index is said to be more useful for diagnosis of a histamine intolerance syndrome.

US 2008/227116 A1 and Appl Biochem Biotechnol 2007 Nov;143(2):164-75 describe a method wherein the DAO in a sample is captured by an immobilized antibody. A diamine substrate is added to the immobilized DAO and reacted which gives rise to hydrogen peroxide as side product. The formed hydrogen peroxide can be determined via the oxidation of a luminescence reagent and assumed to be proportional to the amount of active DAO. This assay turned out not dependable for various experimental drawbacks. US 5,124,254 A (Hewlins et al) describes a similar diagnostic kit. The DAO-produced hydrogen peroxide is be determined by its oxidation of a chromogenic system or *in situ* by a peroxidase-based sensor (Hibi T et al, Enzymatic assay of histamine by amperometric detection of H202 with a peroxidase-based sensor, Biosci Biotechnol Biochem 2000, 64(9):1963-1966).

Further background with respect to the above can be found in:-

Kehoe CA et al., Plasma diamine oxidase activity is greater in copper-adequate than copper-marginal or copper-deficient rats, J. Nutr 2000, 130:30-33;
Küfner MA et al, Total histamine degradation capacity (THDC) as an important biological marker of histamine metabolism in human colonic mucosa, Inflamm Res 2002, Suppl 1:87-81;
Nilsson B-O et al., Inhibition of diamine oxidase promotes uptake of putrescine from rat small intestine, Inflamm Res 1996, 45:513-518;
Novotny et al., Diamine oxidase is the amiloride-binding protein and is inhibited by amiloride analogues, J Biol Chem 1994, 269:9921- 9925;
Oguri S et al., Direct detection of endogenous histamine in rat peritoneal mast cells by in-capillary derivatization high-performance capillary electrophoresis, J Chromatogr B Biomed. Sci Appl 1999, 736:263-71;
Pietrangeli P et al., Inactivation of copper-containing amine oxidases by turnover products, Eur J Biochem, 2004, 271:146-152.

The current methods for as well as the diamine/histamine conversion ratio and a diamine/histamine conversion rate per unit time diagnosis of histamine intolerance syndrome are all based on a measurement of serum DAO or DAO activity in blood, plasma, or serum. The state of the art therefore represents a problem.

## BRIEF DESCRIPTION OF THE INVENTION

The invention is defined in the appended claims. The problem is solved by a method of determining the histamine inactivation in a human subject suspected of suffering from histamine intolerance syndrome and/or insufficient DAO enzyme activity comprising an administration of a known amount of stable isotope-labeled histamine and measuring isotope-labeled products of the human histamine degradation pathways using a common derivatization and an LC-MS/MS technique. Alternatively, a serum or plasma sample is taken wherein the histamine inactivation is to be examined and subjected to physiological conditions after addition of a predefined amount of stable isotope-labeled histamine and followed by measuring isotope-labeled products of the human histamine degradation pathways using an LC-MS/MS technique after a common one-pot derivatization of the carboxyl groups of common histamine degradation products.

In some embodiments, the method of differential diagnosis of histamine intolerance syndrome comprises the

steps of: (i) administering a subject suspected of suffering from insufficient histamine degradation and/or DAO enzyme activity a histamine load containing a known amount of stable isotopic labeled histamine; (ii) obtaining a sample of bodily fluid selected from blood, serum, plasma, urine, or saliva after a predefined period of time, optionally followed by a removal of proteins; (iii) chemical derivatization of the metabolites of histamine using a derivatization agent which reacts with the carboxyl group of imidazole acetic acid and N-methyl-imidazole acetic acid; (iv) measuring the derivatized products contained in said sample of bodily fluid using a LC-MS/MS technique; and (vi) calculating the subjects' histamine inactivation activity on basis of selected stable-isotope labeled fragments of imidazole acetic acid and methyl imidazole acetic acid.

The stable isotopic labeled histamine may be selected from histamine $^{15}$N-labeled at positions N1 and/or N3, histamine $^{13}$C-labeled at positions C2, C4, C5, C6, or C7, histamine deuterium labeled at any hydrogen position, or a combination of the aforementioned isotopic labels.

In some embodiments, the method may further comprise an immunological determination of the amount of soluble human diamine oxidase in serum or plasma.

The reagents for an activation of the carboxyl group of imidazole acetic acid and methylimidazole acetic acid for derivatization may be selected from (1) triphenylphosphine (TPP), diphenyl-2-pyridylphosphine, tributylphosphine (PBu3), 1-ethyl-3-(3-dimethylaminopropyl)carbodiimid * HCl (EDC), 4-dimethyl(aminophenyl)diphenyl-phosphine, 4-(dimethyl amino)phenyl phosphine, tris(4-dimethylaminophenyl) phosphine in combination with (2) 2,2'-dipyridyldisulfide (DPDS), dimethyl acetylenedicarboxylate (DMAD), diisopropyl azodicarboxylate (DIAD), di-tert-butyl azodicarboxylate (DTBAD), diethyl azodicarboxylate (DEAD), dimethyl azodicarboxylate, N,N'-dicyclohexylcarbodiimid (DCC), N-hydroxysuccinimide (NHS), N-hydroxysulfosuccinimide (sulfoNHS). The activating reagents under point (1) may be used without a second activating agent from group (2) but this may result in an incomplete derivatization reaction.

The functional derivatization agent may be selected from the group comprising aromatic hydrazine compounds, hydrazinoquinoline, 2-hydrazinoquinoline, substituted hydrazinoquinoline, 7-chloro-4-hydrazinoquinoline, dialkyl hydrazinoquinoline, 3,8-diethyl-2-hydrazinoquinoline, 6-fluoro-4-hydrazinoquinoline, 2-hydrazinopyridine, phenylhydrazine, or aromatic primary amines, 2-picolylamine, 4-(N,N-dimethylaminosulfonyl)-7-piperazino-benzofurazan (DBD-PZ), 6-aminoquinoline, 4-(2-((4-bromophenethyl)dimethylammonio)-ethoxy)benzenaminium dibromide (4-APEBA), 4-(2-(trimethylammonio)ethoxy)-benzenen-aminium dibromide (4-APC), 4-bromo-N-methylbenzylamine (4-BNMA), 3-methyl-N-methylbenzylamine (3-BNMA), 4-bromo-n-methylaniline (4-BMA). A person skilled in the art may of course contemplate other suitable derivatization reagents on basis of the provided technical principle.

Another aspect concerns a method of *in-vitro* diagnosis of histamine intolerance syndrome, comprising the steps of: (i) obtaining a sample of plasma or serum from a patient suspected of suffering from histamine intolerance; (ii) adding to said sample a predefined amount of stable isotope-labeled DAO substrate to obtain a defined concentration of isotope-labeled DAO substrate in said sample; (iii) incubation of said sample under physiological conditions for a predefined period to obtain degradation of said isotope-labeled substrates and chemical derivatization of the carboxyl group containing degradation products of histamine; and (iv) determining by mass spectrometry, which comprises a LC-MS/MS technique, the concentration of derivatized degradation products of said isotope-labeled DAO substrate to calculate a histamine conversion ratio or a conversion rate per unit time for comparison with the histamine conversion ratio and conversion rate found in a sample from a healthy subject and diagnosis of histamine intolerance when said conversion is low.

In some embodiments, the method may include a spiking of the serum or plasma sample with an isotopic labeled diamine/histamine to achieve a spiking concentration from 10 nM (final concentration sample e.g. histamine 1,84 ng/mL) to 50 $\mu$M litre-1 (final concentration sample, e.g. histamine 9200 ng/mL) serum, while a most preferred spiking concentration is 200 nM (histamine 36,8 ng /mL).

In some embodiments, the method may comprise a calculating of the activity of soluble human DAO enzyme in said sample at given physiological conditions from the amounts of DAO substrates converted per unit of time and a determining of the half-life of histamine and/or DAO substrate in said sample, to diagnose histamine intolerance when the patient's diamine oxidase activity is below 3 enzyme units (U) per millilitre serum or plasma or below 10 enzyme units (U) per millilitre as measured by the immunoassay and/or the half-life of labeled histamine in serum is above a certain threshold (range 2 minutes to 24 hours, depending on the spiked histamine concentration), the latter being dependent on the specific physiological conditions. In some embodiments, the method further comprises an immunological determination of soluble human diamine oxidase in serum or plasma and a determination of the enzyme activity of DAO in serum or plasma.

Another aspect relates to a kit for *in-vitro* diagnosis of histamine intolerance syndrome, comprising a defined histamine preparation as oral load, preferably in the form of a solution, dispersion or tablet, which contains a defined amount of stable isotopic labeled histamine selected from histamine $^{15}$N-labeled at positions N1 and/or N3, histamine $^{13}$C-labeled at positions C2, C4, C5, C6, C7 or a histamine deuterium labeled at any hydrogen position, or a combination of said isotopic labels; and reagents for activation and derivatization of imidazole acetic acid and N-methyl imidazole acetic acid, preferably comprising any one of hydrazinoquinolines, 2-hydrazinoquinoline, substituted hydrazinoquinoline,

4-hydrazinoquinoline; 7-chloro-4-hydrazinoquinoline, dialkyl-hydrazinoquinoline, 3,8-diethyl-2-hydrazinoquinoline, 6-fluoro-4-hydrazinoquinoline; as well as buffers and solutions for sample preparation.

Alternatively, the kit for *in-vitro* diagnosis of histamine intolerance syndrome may be one for measurement in a sample of plasma or serum. The histamine spiking solution or histamine load may contain a defined amount of stable isotopic labeled histamine as shown in formula I, selected from group (1) histamine $^{15}N$-labeled at positions N1 and/or N3 or group (2) histamine $^{13}C$-labeled at positions C2, C4, C5, C6, or C7 or group (3) histamine deuterium labeled at any of the hydrogen positions, or a combination of the aforementioned isotopic labels. The derivatization agent for derivatization of imidazole acetic acid and N-methyl imidazole acetic acid is chosen from the group comprising derivatized or substituted hydrazinoquinolines, 2-hydrazinoquinoline, 4-hydrazinoquinoline. The kit may further comprise buffers and solutions for sample preparation and activation of the carboxy group of imidazole acetic acid and methyl imidazole acetic acid. In some embodiments, the kit may further comprise standards of imidazole acetic acid and methyl imidazole acetic acid which are additionally isotopic labeled at least at one more position. In some embodiments, the kit may further comprise two to six matrix standards with varying concentrations of a mixture of the expected isotopic labeled imidazole acetic acid and methyl imidazole acetic acid.. In some other embodiments, the kit may further comprise an enzyme solution of catalase, peroxidase, and/or alcohol dehydrogenase, preferably adapted for incorporation into serum or plasma.

The isotopic labeled histamine may be labeled at the positions shown in the formula I. Preferred educts should be labeled *at least two* of the given positions with the corresponding heavy isotope:

(histamine)

Formula I

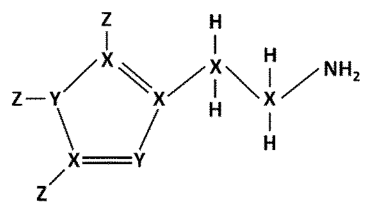

$X = {}^{13}C$ $Y = {}^{15}N$ $Z = {}^{2}H$ (D)

The resulting isotopic labeled imidazole-4-acetic acid and N-methyl imidazole-4- acetic acid will therefore be of formulae II and III below: -

(imidazole-4-acetic acid)

Formula II

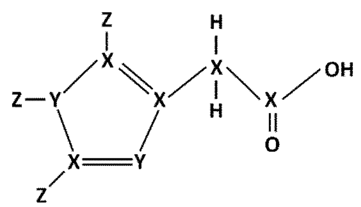

$X = {}^{13}C$ $Y = {}^{15}N$ $Z = {}^{2}H$ (D)

(N-methyl imidazole-4-acetic acid)

Formula III

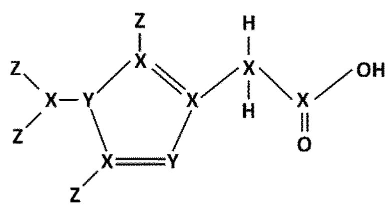

$X = {}^{13}C$ $Y = {}^{15}N$ $Z = {}^{2}H$ (D)

The crux of the method is that histamine is inactivated in the body intracellularly by the enzyme histamine N-methyltransferase (NMT) and extracellularly by the enzyme diamine oxidase as well as by enzymes of the monoaminooxidase (MAO) family. As shown in Figure 1, the histamine inactivation pathways merge - regardless of the inactivation by NMT, secreted DAO, membrane-associated DAO or any MAO - at the carboxyl group containing metabolites of formulae II and III (imidazole-4-acetic acid and N-methyl-imidazole-4-acetic acid). These metabolites can be chemically derivatized *in situ* in a one-pot reaction and thereafter be analysed and quantified using a LC-MS/MS technique. The one-pot derivatization is for improved LC separation and preconcentration performance and increased detector response in the LC-MS/MS technique as suitable functional derivatization groups lead to higher detector signals (see Figures 4 to 7). The various inactivation pathways of histamine can therefore be analysed by examining a limited number of isotopic labeled fragments of derivatized oxidized histamine products by MS/MS. The method allows a following up and simultaneous detection of the degradation products of histamine (His) and N-methyl histamine (MetHis) which are N-methyl imidazole acetic acid (Mimi) produced in the intracellular inactivation and degradation pathway (1) and imidazole acetic acid (Imi) produced by the extracellular inactivation and degradation pathway (2). It also takes account an inactivation of histamine by enzymes of the monoaminooxidase family. Histamine and methylhistamine could be separated using hydrophilic interaction chromatography (HILIC). HILIC uses hydrophilic stationary phases with reversed-phase type eluents so that analytes elute in the order of increasing polarity. This does not work well for the degradation products, say for imidazole-4-acetic acid and methyl imidazole-4- acetic acid, which are therefore subjected a one-pot derivatization as described to allow separation on a reverse phase column.

The carboxyl groups of imidazole acetic acid or N-methyl-imidazole acetic acid can be activated and derivatized. A preferred derivatization reagent is 2-hydrazinoquinoline (2-HQ) as the resulting isotopic labeled 2-HQ derivatives of formulae IV and V are for optimized separation and detection by LC-MS/MS:

(2-HQ-Imidazole acetic acid)

Formula IV

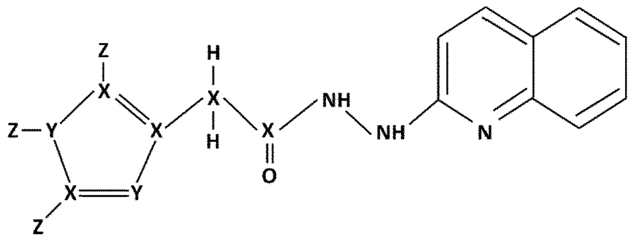

X = $^{13}C$ Y = $^{15}N$ Z = $^{2}H$ (D)

(2-HQ-N-methyl imidazole acetic acid)

Formula V

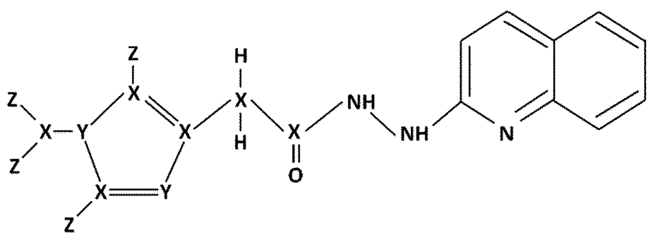

X = $^{13}C$ Y = $^{15}N$ Z = $^{2}H$ (D)

Another aspect concerns a differential *in-vitro* diagnosis of histamine intolerance syndrome, comprising the steps of:-

obtaining an aliquot of plasma or serum from a patient suspected of suffering from histamine intolerance syndrome;
determining the concentration of human diamine oxidase (hDAO - EC 1.4.3.22) in said aliquot using an immunoassay for human diamine oxidase (DAO);
mixing (spiking) an aliquot of plasma or serum with a predefined amount of DAO substrate selected from stable-isotope labeled histamine or putrescine (1,4-diaminobutane) or cadaverine (1,5-diaminopentane) at a concentration range of from 10 to 10000 nM (nanomolar), preferably from 100 to 500 nM,
incubation of the spiked sample at physiological conditions (e.g. pH 7.2 to 7.8, preferably 7.3 to 7.6; 36-38°C) for a predefined period from 2 minutes to 24 hours; and
determining by a combination of liquid chromatography and mass spectrometry in said aliquot the concentrations of oxidized metabolites;
to calculate the activity of DAO and/or the diamine/histamine conversion ratio and/or the diamine/histamine conversion rate per unittime in serum or plasma of a patient suspected of suffering from histamine intolerance syndrome, and to diagnose a histamine intolerance when the patient's DAO activity in said sample is below 3 enzyme units (U) per millilitre serum at physiological conditions of the blood and/or below normalized 10 enzyme units (U) per millilitre or the calculated half-life of dietary histamine is above a certain threshold (2 minutes to 24 hours depending on substrate and concentrations)

The application further contemplates a determination of the histamine concentration in said aliquot, optionally in addition to the theoretical concentration of added histamine. Using the diamine conversion ratio and diamine conversion rate as determined from the oxidized histamine metabolites the specific histaminase activity in serum can be determined.

A further aspect relates to an *in-vitro* determination of the histamine clearance (diamine clearance) in plasma or serum comprising the steps of (a) collecting venous blood in a sampling tube; (b) mixing said serum or plasma sample with a predefined amount of isotopic labeled DAO substrate, preferably isotopic labeled histamine, the amount of spiked isotopic labeled DAO substrate being in a concentration range of from 10 to 1000 nmol·litre-1, preferably 100 to 500 nmol·litre-1; (c) incubation of the DAO substrate-spiked sample at physiological conditions for a predefined period of from 2 minutes to 24 hours to obtain oxidative degradation of the isotope-labeled histamine or diamine by the activity of the human DAO enzyme contained in said sample; (d) derivatization of the metabolites and application of said sample to LC-MS/MS to determine the concentrations of isotopic labeled degradation product to determine the diamine/histamine clearance in said sample.

Another aspect relates to an *in-vitro* determination of the total histamine clearance capacity (total diamine clearance) in plasma or serum, comprising the steps of (a) collecting venous blood in a sampling tube; (b) mixing said blood sample with a predefined amount of stable-isotope labeled DAO substrate, preferably isotopic labeled histamine, the amount of spiked isotope-labeled DAO substrate being in a concentration range of from 10 to 10 000 nmol·litre-1, preferably 100 to 500 nmol·litre-1; (c) incubation of the DAO substrate-spiked sample at physiological conditions for a predefined period of from 2 minutes to 24 hours to obtain degradation of the isotopic labeled histamine or diamine by the activity of the human DAO enzyme contained in said sample; (d) derivatization of the metabolites and application of said sample to LC-MS/MS ***to*** determine the concentrations of either the non-degraded stable isotope-labeled histamine or diamine in said serum sample and/or isotope-labeled degradation products to determine the total diamine or histamine clearance in said sample.

A further aspect relates to a kit comprising (i) reagents for determining the concentration of DAO in blood, serum or plasma by an immunoassay, (ii) one or more columns, preferably a solid phase extraction column, for use in a deproteinization and/or prepurification of the sample and (iii) reagents for derivatization prior liquid chromatography and mass spectrometry.

The invention will be further described with respect to its advantages, embodiments, specific examples, and drawings which shall not be considered limiting. Reference to any prior art in the detailed description is not an acknowledgement or suggestion that this prior art forms part of the common general knowledge or that this prior art could reasonably be expected to be combined with any other piece of prior art by a skilled person in the art. By way of clarification and for avoidance of doubt, as used herein and except where the context requires otherwise, the term "comprise" and variations of the term, such as "comprising", "comprises" and "comprised", are not intended to exclude further additions, components, integers or steps. The scope of the invention is described in the claims.

## BRIEF DESCRIPTION OF DRAWING

It is shown in

Fig. 1 a schematic representation of the two histamine inactivation pathways in the body;

Fig. 2 diagrams of a chromatographic separation of 2HQ-imidazole acetic acid (Imi) and 2HQ-methyl imidazole acetic acid (Met) in plasma and serum using a reversed phase column;

Fig. 3 comparative chromatographic separations of 2-HQ-imidazole-4-acetic acid/2HQ-methylimidazole-4-acetic acid and 2-HQ-$^{13}$C5-imidazole-4-acetic acid/2-HQ-$^{13}$C5-methylimidazole-4-acetic acid in serum;

Fig. 4 a representative full scan MS/MS spectrum of 2HQ-imidazole acetic acid;

Fig. 5 a representative full scan MS/MS spectrum of 2HQ-$^{13}$C5-imidazole-4-acetic acid;

Fig. 6 a representative full scan MS/MS spectrum of 2-HQ-methylimidazole-4-acetic acid;

Fig. 7 a representative full scan MS/MS spectrum of 2-HQ-$^{13}$C5-methylimidazole-4-acetic acid;

Fig. 8 a diagram of a linear regression for determining the sensitivity for 2-HQ-imidazole-4-acetic acid as calculated over the calibration range of 1 to 100 ng/mL (increments 1, 2, 5, 10, 20, 50 and 100 ng/mL) in serum:

Fig. 9 a diagram of a linear regression for determining the sensitivity for 2-HQ-methylimidazole-4-acetic acid as calculated over the calibration range of 1 to 100 ng/mL (increments 1, 2, 5, 10, 20, 50 and 100 ng/mL) in serum.

## DETAILED DESCRIPTION OF THE INVENTION

Histamine is present in many fermented foods and beverages and high levels of ingested histamine can trigger pseudoallergic reactions, asthmatic wheezing, swelling of eyelids, eye turgor and respiratory mucosa, flushing, skin reddening, rushes, urticaria, pruritus. High levels of ingested histamine may also lead to a lowering of the blood pressure, contraction of the smooth muscles in the intestine and respiratory tract, and therefore to gastrointestinal problems, abdominal pain, constipation, diarrhoea, migraine, headache, arrhythmia, oedema and sleep disturbances.

Histamine intolerance syndrome is observed in cases of increased availability of histamine or impaired histamine degradation or both. An increased availability of histamine may be due to an overproduction of endogenous histamine under conditions of allergies, mastocytosis, bacterial infections, gastrointestinal bleedings and/or due to increased ingestion of histidine or histamine by a consumption of fermented food, often in combination with a consumption of alcohol. Alcohol and histamine metabolic pathways have the common enzymes aldehyde dehydrogenase and aldehyde oxygenase and a consumption of alcohol will therefore enhance the physiological effects of histamine (Zimatkin SM et al, Alcohol-histamine interactions, Alcohol Alcohol 1999, 34(2):151-7). Histamine degradation may be inhibited or compromised by a lack of co-factors such as vitamin C, vitamin B6, copper or manganese ions or the presence of competing biogenic diamines. It is further known that the activity of DAO in serum is inhibited by numerous drugs, including muscle relaxants (pancuronium, alcuronium, D-tubocurarine), narcotics (thiopental), analgetics (morphine, pethidine, non-steroidal and anti-inflammatory drugs (acetylsalicylic acid, metamizole), local anaesthetics (prilocaine), antihypotonics (dobutamine), antihypertensive drugs (verapamil, alprenolol, dihydralazin), antiarrhythmics (propafenone), diuretics (amiloride), drugs influencing gut motility (metoclopramide), antibiotics (cefuroxime, cefotiam, isoniazid, pentamidin, clavulanic acid, chloroquine), mucolytics (acetylcysteine, ambroxol), broncholytics (aminophylline), H2-receptor antagonists (cimetidine), cytostatics (cyclophosphamide), antidepressants (amitriptyline). DAO levels in humans further increase sharply within 30 minutes of heparin administration while the presence of heparin in experiments with cell culture has no impact on the DAO level or its activity.

There are two primary pathways for histamine inactivation: - (i) by methylation of its imidazole ring which is catalysed by histamine-N-methyltransferase (NMT). The resulting N-methylhistamine is physiologically not active. And, (ii) by oxidative deamination of the primary amino group of histamine catalysed by diamine oxidase (DAO). NMT in the cytosol controls the transmission of the histaminergic signals in the nervous system and DAO in serum or tissue-bound (e.g. intestinal mucosa, kidney) degrades the dietary histamine when passing into the circulation and thereafter.

The symptoms of histamine intolerance are only observed when histamine levels reach in serum a pathological level of 9 nmol (about 1 ng /mL serum) or above. The histamine levels in serum are however not *directly* dependent on the amount of DAO enzyme in the blood or its activity. As foods and beverages may contain histamine in amounts of up to 50 mg per 100 grams, their consumption corresponds to a spiking of the physiological histamine clearance capacity. The spiking of a plasma or serum sample with isotopic labeled histamine ("diamine-spiking") challenges the histamine clearance. A measured DAO activity of 3 enzyme units in serum would then correspond to a clearance of 3000 nmol histamine per minute. Pathological histamine concentrations of up to 5 to 10 000 nmol·litre-1 serum have been determined in patients with anaphylactoid reactions. Thus, a measured DAO activity in serum of 3 enzyme units as determined by a spiking experiment would corresponds to a half-life of less than 2 minutes to 4 hours for highly increased histamine levels, e.g. as would be obtained after the normal consumption of non-toxic food and beverages high in histamine. Thus, a DAO activity in serum of 3 enzyme units or more excludes any symptoms of histamine intolerance.

The measured DAO activity following a spiking of a plasma or serum sample with a histamine challenge should not be confused with a measurement of the statutory DAO in serum as disclosed by prior art methods. The metabolic pathways of histamine and alcohol are linked because the primary metabolite of ethanol degradation, acetaldehyde, and the primary metabolites of histamine degradation, methylimidazole acetaldehyde and imidazole acetaldehyde, compete for the same aldehyde dehydrogenase and aldehyde oxidase. Alcohol and acetaldehyde further induce a release of histamine from the storage granules in peripheral mast cells so that a consumption of alcohol can provoke food-induced histaminosis which leads to gastric and intestinal damages and bronchial asthma in many persons. An increasing epidemiological problem. Histamine levels in common alcoholic beverages range from 3 to 120 micrograms/L in white wine, 20 to 300 micrograms/L in beers, from 15 to 700 micrograms/L in champagnes and from 60 to 3800 micrograms/L in red wine.

If the measured half-life of "spiked" (dietary) histamine in serum is below a threshold (120 seconds to 4 hours, depending on circumstances) the patient will likely not suffer from increased histamine levels and this will also apply to a normal consumption of foods rich in histamine. On the other hand, we now know that a measurement of the statutory DAO or its activity in serum allows no reliable differentiation between histamine intolerance and e.g. a food allergy. A conventional determination of a diamine/histamine conversion rate in serum may therefore be not enough to diagnose histamine intolerance or the aetiology of histamine intolerance and why patients are suffering from an unphysiological histamine boost over an extended period of time (several hours or overnight).

The patient's total capacity for ingested histamine clearance (tissue associated DAO and secreted serum DAO) must be known for differential diagnosis of histamine intolerance from food hypersensitivity, food intolerance and food allergy and this non-compromised (not inhibited by alcohol or drugs) total dietary histamine clearance requires a measurement of histamine metabolites in bodily fluids. This is because the amount and activity of tissue-associated DAO of a patient also contributes to the total dietary histamine clearance capacity. In the prior art, the serum DAO activity was never measured having regard to the total clearance of ingested histamine which involves tissue-associated DAO. The prior art methods merely determined which histamine clearance is achieved in the serum by an addition external diamine (histamine or putrescine). In other words, the measurements were done with the expectation that the soluble DAO activity in serum would coincide with the total amount of DAO enzyme in the body. That model did not consider the various histamine inactivation pathways nor the multiple forms of DAO.

The present invention considers that the metabolic pathways may change by an excess of dietary histamine, that histamine degradation in serum can be compromised by the presence of drugs or alcohol and that serum DAO activity is dependent on multiple factors, including pH because histamine may also be a substrate of monoamine oxidases (MAO) at higher pH above 8.0. Prior art assays even recommend using a Tris-HCl buffer (tris-(hydroxymethyl)-aminomethane) having a pH of 8.0 to 8.5 for a determining the diamine/histamine conversion ratio and rate as such a buffer solution has "particularly positive effects on the DAO activity" (see US 8 003 343, column 5). The conventional assays for diagnosis of histamine intolerance never examined or quantified the "inactivated" metabolites of histamine and could not differentiate between monoamine oxidase (MAO) activity and diamine oxidase (DAO) activity nor detect any inhibition, e.g. by external drugs or a lack of co-factors.

The present application considers that the DAO Michaelis constants, the Km values of histamine and putrescine (1,4-diamine butane) are about 2.8 micromole and 20 micromole, respectively, as measured using isolated recombinant human kidney DAO expressed in Drosophila cells (see Elmore BO et al., Human kidney diamine oxidase: heterologous expression, purification, and characterization, J Biol Inorg Chem 2002, 7(6):565-579). Thus, the substrate concentrations for histamine and putrescine at which the DAO reaction rate is half of the maximum reaction rate differ by a factor of 7. Putrescine can therefore not be used in methods of diagnosis of histamine intolerance syndrome. Human serum DAO is further partly inhibited by 100 micromolar histamine in the assay and a histamine concentration of 500 micromoles inhibits human DAO activity by about 60% (see US 8,003,343). The present disclosure therefore considers for as well as the diamine/histamine conversion ratio and a diamine/histamine conversion rate per unit time diagnosis of histamine intolerance in accordance with the Michaelis-Menten model for catalysts in biological system that the DAO enzyme (E) must first combine with the diamine substrate (S) to form an enzyme-substrate (ES) complex. This enzyme-substrate (ES) complex can then proceed with the oxidative degradation of the diamine/histamine and dissociate again. Thus, the degradation rate of histamine is given by the equation

$$\mathrm{V} = V\max \frac{[S]}{[S] + K(M)}$$

in which Vmax is the degradation rate when the DAO enzyme is fully saturated with substrate and Km, the Michaelis-Menten constant, is the substrate concentration at which the reaction rate is half maximal and [S] is the concentration of the substrate.

The prior art works with an excess of histamine or diamine in the assay and ignores the different Km values for histamine and putrescine so that no more than a maximal DAO activity at an artificial "pH and enzyme environment" is determined which is further compromised by competing metabolic pathways and varying amounts of histamine, diamine, DAO and/or MAO in the sample. Prior art solutions for measuring the DAO enzyme activity work with histamine or diamine in an amount of about 80 to 100 micromoles per litre, claiming a range from 5 to 400 micromoles, which is

well above physiological levels. The usual limit for pathological histamine values in serum is 9 nanomoles per litre. Histamine concentrations in serum of patients with an anaphylactoid reactions are below 10 000 nanomoles (nmol) per litre (L), say less than 10 micromolar, with histamine disappearing from serum at 37°C with an apparent half-life of 2 minutes to 4 hours. Thus, a theoretical maximal DAO activity is determined or a wrong activity but not the actual DAO enzyme activity in serum. On the other hand, the present method allows an administration or oral administration of typical dietary or physiological concentrations of histamine due to the high sensitivity of the LC-MS/MS detection method. The resulting serum levels of isotopic labeled imidazole acetic acid and methyl imidazole acetic acid, as well as of other histamine metabolites take account of the activity of tissue-associated DAO, e.g. in the mucosal membrane and in the kidney, in addition to serum DAO.

The kinetic studies on the deaminating oxidation of $^{14}C$-putrescine at concentrations from 1 micromole to 5 millimole confirm the existence of two enzymes in serum: DAO with high affinity which is sensitive to alpha-aminoguanidine, and MAO with a lower affinity for the substrate which is selegiline-sensitive. The present disclosure refers to a method of diagnosing histamine intolerance when the diamine oxidase activity in serum is below 3 enzyme units (U) per millilitre serum, if determined *in vitro* at RT (25°C). The disclosure further comprises a method of diagnosis wherein the actual diamine oxidase activity in serum is 50% of the normalized diamine oxidase activity in serum as determined by an immunoassay for human DAO, indicating an inhibition of the enzyme DAO in serum by the presence of drugs or other biogenic amines.

The serum DAO concentration and the measured serum DAO activity by REA are already used biomarkers for histamine intolerance and associated symptoms. Both, serum DAO and the measured DAO activity in serum complement each other as the DAO activity in serum depends on the absence of inhibitors (drugs, alcohol) and cofactors such copper ions, manganese ions and vitamin C or vitamin B6. By further quantitating the cofactors in blood, the clinical laboratory will allow a treatment of the disorder in respect of the co-factors which have to be supplemented or which drug should be better replaced in order not to inhibit dietary histamine degradation. A differential diagnosis of histamine intolerance syndrome vis-à-vis food intolerance, food hypersensitivity or food allergy is not possible.

Histamine intolerance (HIT) occurs when the combined activities of serum DAO and tissue-associated DAOs in the intestinal mucosal membrane, kidney, lungs are not enough to degrade a dietary histamine spike in addition to the histamine produced in the body. The overall histamine degradation capacity in the body depends likewise on the absence of DAO inhibitors and the presence of co-factors. The large sequence identity between secreted DAO and tissue-associated DAOs suggests that the inhibitors and co-factors affect most similar the overall histamine degradation capacity in the body as can be determined for the degradation capacity of secreted serum DAO alone. The specific enzyme activity of DAO in a patient's sample is therefore a reference measure of the overall histamine degradation ability in the body.

The specific enzyme activity is the amount of product formed in each amount of time under given conditions per milligram of enzyme. The specific activity is equal to the rate of reaction multiplied by the volume of reaction divided by the mass of total protein. The SI unit is the katal, 1 katal = 1 mol s-1, but this is an excessively large unit. A more commonly used value is the (one) enzyme unit (U) which is 1 micromole min-1. (One enzyme unit (U) corresponds to 16.67 nanokatals). The specific enzyme activity is therefore a measure of the processivity at a specific (usually saturating) substrate concentration. Where the molecular weight and the precise amount of the enzyme is not known, as in the present case, the measured turnover of isotope-labeled histamine in serum and the separately measured specific activity of serum DAO allow calculation of how much DAO enzyme, secreted or tissue-associated, is effectively present in the body and its total activity for degradation of dietary histamine taken up with the foods. The turnover number can be visualized as the number of times each enzyme molecule conducts its catalytic cycle per second. The method of the invention therefore allows a reliable differentiation between histamine intolerance syndrome and food intolerance or food allergy. The histamine intolerance syndrome goes in line with an insufficient degradation of a spike of dietary histamine for a lack of tissue-associated DAO in the intestinal mucosal membrane and to a minor degree for a lack of serum DAO. The symptoms of food allergy or food hypersensitivity are caused by a spike of endogenous histamine.

Gas chromatography-mass spectrometry (GC-MS) has been an analytical platform for detecting polar ketones, aldehydes, carboxylic acids because of its high resolution and sensitivity. The derivatization methods for GC analysis comprise a tandem oximation-silylation procedure. The oximation of aldehydes and ketones is achieved by a reaction between the carbonyl group and methoxyamine. The reaction with silylation agents replaces the active hydrogen in alcohols, carboxylic acids, amines, and thiols. Both reactions for a GC-based analysis are incompatibility with water. The necessary dehydration by solvent extraction, evaporation, or lyophilization makes the sample preparation for GC-MS analysis time-consuming and the entire process is therefore too inefficient for a use in a clinical laboratory. Thus, a method for liquid chromatography-tandem mass spectrometry (LC-MS/MS) was developed which is more compatible with bodily fluids. A direct LC-MS/MS analysis of histamine and its degradation/inactivation products of histamine is difficult due to their poor chromatographic performance and low ionization efficiency. The reverse phase LC column are not well suited for retaining and separating these hydrophilic metabolites, leading to poor or no signals in the mass detector. The hydrophilic interaction liquid chromatography (HILIC) offers improved chromatographic separation of polar

compounds. On the other hand, a chemical derivatization leads to a better chromatographic performance and detectability of the histamine degradation products: imidazole acetic acid and N-methylimidazole acetic acid. Esterification and amidation are typical derivatization reactions for carboxylic acids. The functional groups 2-picolylamine and 2-hydrazinopyridine give improved ionization efficiency of carboxylic acids (Higashi T et al, Simple and practical derivatization procedure for enhanced detection of carboxylic acids in liquid chromatography-electrospray ionization-tandem mass spectrometry. J Pharm Biomed Anal 2010, 52: 809-818) Other derivatization reagents for carboxylic acids include 2-chloro-1-methylpyridinium (Cartwright AJ et al., Derivatisation of carboxylic acid groups in pharmaceuticals for enhanced detection using liquid chromatography with electrospray ionisation tandem mass spectrometry. Rapid Commun Mass Spectrom 2005, 19:1058-1062), triethylamine (Barry SJ et al., Derivatisation for liquid chromatography-/electrospray mass spectrometry: synthesis of pyridinium compounds and their amine and carboxylic acid derivatives. Rapid Commun Mass Spectrom 2003, 17:603-620), and heptadecafluoroundecylamine (Hayama T et al., Fluorous derivatization combined with liquid chromatography/tandem mass spectrometry: a method for the selective and sensitive determination of sialic acids in biological samples. Rapid Commun Mass Spectrom 2010, 24, 2868-2874). Derivatives of phosphonium bromide, hydroxylamine and hydrazines such as 2,4-dinitrophenylhydrazine and dansyl hydrazine lead to derivatives which can be more easily ionized than the parent compounds. A most preferred hydrazine derivatization agent are hydrazinoquinolines, in particular, 2-hydrazinoquinoline, as they allow a derivatization of the oxidized histamine metabolites in urine, serum, plasma and tissue extracts in one hour. Compared to derivatives of 2-hydrazinopyridine, 2-picolylamine and dansyl hydrazine, hydrazinoquinolines derivatives achieve better chromatographic performance in reversed phase LC system and higher ionization efficiency. The use of 2-hydrazinoquinoline as derivatization agent of substituted and non-substituted imidazole acetic acid for simultaneous LC-MS analysis provides therefore a new platform for determining the total histamine degradation rate in the body.

The isotopic labeled histamine and N-methyl-histamine may be labeled at positions shown in formulae I. Preferred internal standards of the expected histamine metabolites may be labeled additionally, e.g. at least at one more position, preferably at three or four positions with the corresponding heavy isotope.

As shown in Fig. 1, histamine is inactivated intracellularly by histamine N-methyltransferase (NMT) and extracellularly by diamine oxidase (DAO) and enzymes of the monoamine oxidase family (MAO). The histamine inactivation pathways all merge - irrespective of the oxidation by secreted DAO or membrane-associated DAO or any MAO - at compounds of formulae II and III (imidazole-4-acetic acid and N-methyl-imidazole-4-acetic acid) which can be collectively derivatized in a one-step/one-pot reaction for LC-MS/MS analysis. The derivatization gives improved LC separation performance and the introduced functional groups by the derivatization as disclosed lead to higher detector signals. This allows a handling of all histamine inactivation pathways in the analytics and allows a more holistic diagnosis. More precisely, the described method allows to follow up and detect simultaneously all inactivation products of histamine (His), including the initial methylation to N-methyl histamine (MetHis) and the subsequent oxidation to N-methyl imidazole acetic acid (Mimi) of the inactivation/degradation pathway (1) as well as the extracellular inactivation and oxidative degradation to imidazole acetic acid (Imi) of degradation pathway (2). The two imidazole acetic acid products of isotopic labeled histamine as well as histamine could be separated using hydrophilic interaction chromatography (HILIC) coupled to mass spectrometry, while is leads to very long gradients (< 15 minutes) and poor chromatographical resolution for the degradation products.

The present application discloses that the degradation products of histamine - imidazole-4-acetic acid and methyl imidazole-4- acetic acid - can be jointly derivatized - *in-situ like* - by a one-pot derivatization and thereafter be separated on a reverse phase column. Common derivatization reactions include silylation, esterification, acetylation, and alkylation. A suitable derivatization agent is 2-hydrazinoquinoline (2-HQ). Using 2-HQ as derivatization agent the LC-MS/MS method can be optimized for the resulting 2-HQ derivatives shown in formulae IV and V.

The combination of the LC-MS/MS technique, the use of an isotopic labeled histamine for an oral histamine load and/or for spiking and the high analytical sensitivity for the histamine degradation products after a derivatization of imidazole acetic acid and N-methylimidazole acetic acid, this combination allows a differential diagnosis of histamine intolerance syndrome compared to anaphylactoid and allergic reactions because the inactivation of isotopic labeled dietary histamine is determined. The alternative clinical laboratory method may comprise the steps: (i) taking an aliquot of plasma or serum from a patient suspected of suffering from histamine intolerance syndrome; (ii) optionally, determining the concentration of human diamine oxidase (hDAO - EC 1.4.3.22) in said aliquot using an immunoassay for human diamine oxidase (DAO); (iii) mixing (spiking) an aliquot of plasma or serum with a predefined amount of DAO substrate comprising isotopic labeled histamine of formula I at a concentration range of from 10 to 10 000 nanomoles/L, preferably from 100 to 500 nanomoles/L and incubation of the histamine spiked sample at physiological conditions (pH 7.2 to 7.8, preferably 7.3 to 7.6; 36-38°C) for a predefined period from 2 minutes to 24 hours, followed by a chemical derivatization reaction; (iv) transferring said aliquot to a mass spectrometry device, preferably a device comprising a combination of liquid chromatography and tandem mass spectrometry and (v) determining by mass spectrometry in said aliquot the concentrations of histamine metabolites, and optionally of histamine, to obtain a histamine conversion ratio and a histamine conversion rate; to diagnose a histamine intolerance when the patient's DAO activity in said sample is below a

threshold level. This threshold level may be set at 3 enzyme units (U) per milliliter serum at physiological conditions of the blood and/or normalized 10 enzyme units (U) per milliliter. or a half-life of dietary histamine levels of above a certain threshold for healthy subjects (2 minutes to 24 hours, depending on concentrations).

A person skilled in the art will also contemplate to directly determine the histamine concentration in the plasma sample to determine whether it is in a pathological range.

## EXAMPLES

*Example 1 - Representative histamine catabolites and their determination by LC-MS/MS*

For determination and quantitation of representative histamine catabolites 500 μl serum sample and control were transferred into 1.5 ml reaction vials and incubated at 37 degrees Celsius with 100 μl aqueous buffer containing 500 nM (92 ng/mL) isotopic labeled histamine and 1 U/100 μl catalase/peroxidase and aldehyde dehydrogenase for predefined periods (10', 20', 30', 60', 2h, 4h, 6h, 24h).

10 μl sample/control were placed in 0.5 ml reaction vial, mixed with 80 μl acetonitrile, and followed by centrifugation at 9000 x g for 10 minutes. 85 μl supernatant was transferred into a closed reaction vial. The carboxyl groups were activated by 5 μl 200 mM diphenyl-2-pyridylphosphine/2,2-dipyridyldisulfide in acetonitrile. 10 μl 2-hydrazinoquinoline dissolved in acetonitrile was added to a final concentration of 400mM. The derivatization sample was incubated at 60 degrees Celsius for 1,5 hours, the reaction stopped by cooling and addition of 100 μl $H_2O$. The derivatized sample was centrifuged at 9000 x g for 10 minutes and transferred into autosampler vials. 5 μl sample were injected into the LC-MS/MS system.

The baseline separation was achieved on a reversed phase column (Waters ACQUITY UPLC BEH C18 Column, particle 1.7 μm, 2.1 mm X 50 mm - other columns such as C18, C8, C4, PFP and others with varying size and particle sized were also tested). The derivatized sample was separated using two mobile phases. Mobile phase A: 2 % acetonitrile in water. Mobile phase B: 98% acetonitrile. The mobile phases were adjusted at pH 8,2 with 1 mM ammonium acetate, applying a flow rate of 400 μl/min. The column temperature was 40 degrees Celsius (range from 30 - 50 degrees Celsius). The gradient comprised a loading step of mobile phase A for 1 minute (range 0,1 - 2 mins); a slope to 20% mobile phase B within 0.1 minutes, (range 0 - 50% B in 0.1 to 1 mins); a separation slope from 20% B to 60% B in 3 mins; a washout phase with 100% mobile phase B for 1,5 mins (range 95-100% B for 0,5 to 10 mins), a first re-equilibration phase from 100% B to 100% A in 0,1 mins (range time 0,1 to 1 mins) and a second re-equilibration phase with 100% A for 2 mins (range time 0,1 to 10 mins). A skilled person will of course adapt the gradient to the specific LC-system and column. An exemplary gradient (Flowrate: 400 μl/mi; column temperature: 40°C; solvent A: 2% acetonitrile with 1 mM ammonium acetate, pH 8,2; solvent B: 98% acetonitrile with 1 mM ammonium acetate, pH 8,2) is given in Table I below:

TABLE 1

| time [min] | mobile phase B [%] |
|---|---|
| 0 | 0 |
| 1 | 0 |
| 1,1 | 20 |
| 4,1 | 60 |
| 5 | 100 |
| 6,5 | 100 |
| 6,6 | 0 |
| 8,6 | 0 |

The exemplary fragments of Table 2 were used as qualifiers/quantifiers, originated from imidazole-4-acetic acid and methyl imidazole acetic acid. A person skilled in the art can chose alternative fragments (labelled or unlabelled) for unambiguous detection by MS/MS.

2-HQ-imidazole-4-acetic acid

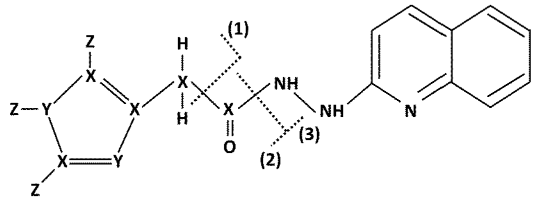

X = $^{13}C$ Y = $^{15}N$ Z = $^{2}H$ (D)

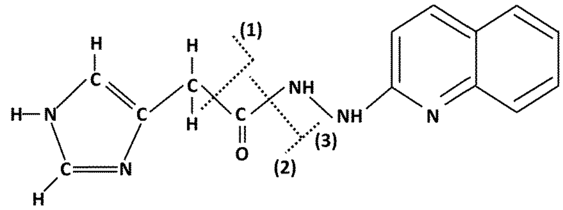

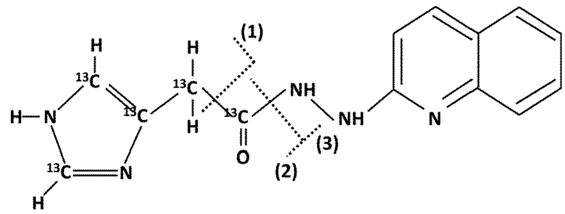

TABLE 2

| Ion | Monoisotopic mass unlabeled [M+H]+ | Monoisotopic mass 13C [M+H]+ |
|---|---|---|
| Precursor | 268,119 | 273,155 |
| Fragment (1) | 81,045 | 85,059 |
| Fragment (2) | 109,04 | 114,058 |
| Fragment (3) | 160,087 | 160,087 |

Fragment (1) imidazole-4-acetic acid

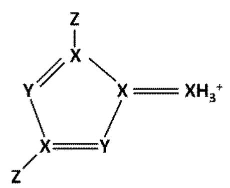

X = $^{13}C$ Y = $^{15}N$ Z = $^{2}H$ (D)

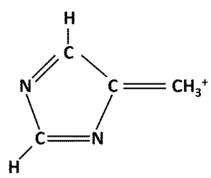

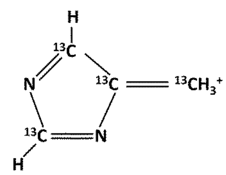

Fragment (2) 2-HQ-imidazole-4-acetic acid

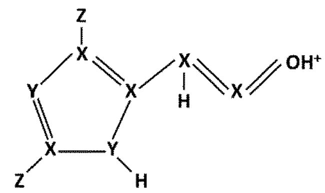

X = $^{13}C$ Y = $^{15}N$ Z = $^{2}H$ (D)

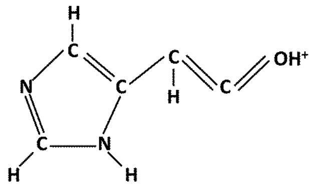

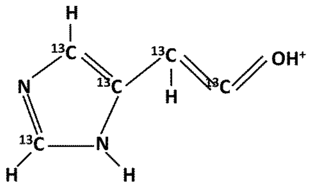

Fragment (3) 2-HQ-imidazole-4-acetic acid

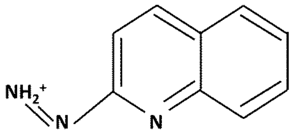

Figure 6 shows an exemplary MS/MS spectrum of unlabelled2-HQ-imidazole-4-acetic acid. Fig. 7 shows an exemplary full scan MS/MS spectrum of 2-HQ-$^{13}C5$-imidazole-4-acetic acid.

2-HQ-$^{13}C5$-methylimidazole-4-acetic acid

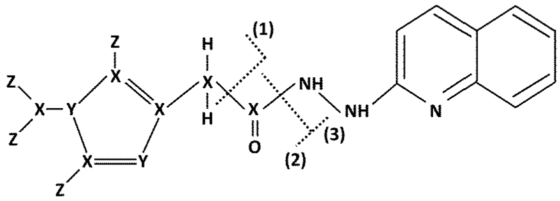

X = $^{13}C$ Y = $^{15}N$ Z = $^{2}H$ (D)

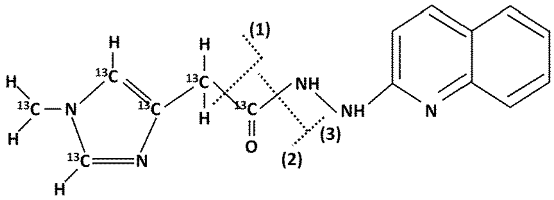

TABLE 3

| Ion | Monoisotopic mass unlabeled [M+H]+ | Monoisotopic mass 13C [M+H]+ |
|---|---|---|
| Precursor | 282,128 | 282,134 |
| Fragment (1) | 95,06 | 99,075 |
| Fragment (2) | 123,055 | 128,073 |
| Fragment (3) | 160,087 | 160,087 |

Fragment (1) 2-HQ-methylimidazole-4-acetic acid

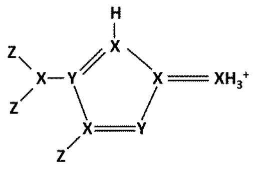

X = $^{13}C$ Y = $^{15}N$ Z = $^{2}H$ (D)

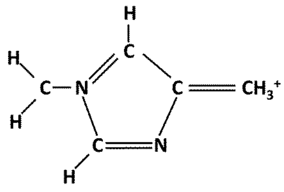

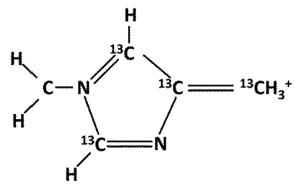

Fragment (2) 2-HQ-methylimidazole-4-acetic acid

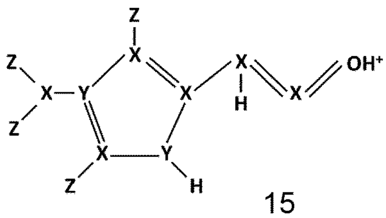

15

X = $^{13}C$ Y = $^{15}N$ Z = $^{2}H$ (D)

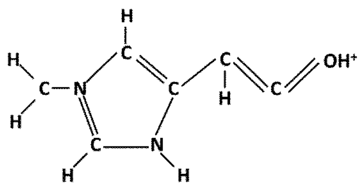

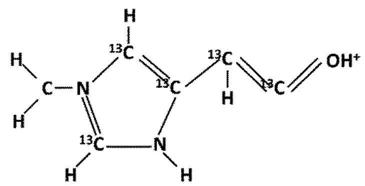

Fragment (3) 2-HQ-methylimidazole-4-acetic acid

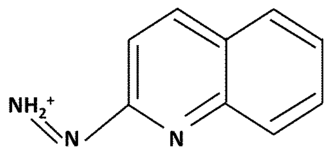

Representative chromatograms of 2-HQ-imidazole-4-acetic acid and 2-HQ-methylimidazole-4-acetic acid from spiked and analysed serum and plasma samples are shown in Figure 2. Figure 3 shows comparative chromatograms of the respective 2-HQ derivatized non-labeled and 2-HQ derivatized isotopic 13C-labeled degradation products.

With respect to 2-HQ-Imidazole-4-acetic acid the described method has a sensitivity of 0,65 ng/mL (average blank signal + 3 standard deviations) and an average accuracy of 101,26 % (± 7,25 %) as calculated over a calibration range of 1 - 100 ng/mL (increments 1, 2, 5, 10, 20, 50, 100 ng/mL, triple injections); see Fig. 8 and linear regression. With respect to 2-HQ-methylimidazole-4-acetic acid the method had a sensitivity of 0,89 ng/mL (average blank signal + 3 standard deviations) an average accuracy of 105,28 % (± 8,09 %) calculated over the calibration range of 1 - 100 ng/mL (increments 1, 2, 5, 10, 20, 50, 100 ng/mL, triple injections); see Fig. 9 and linear regression. All measured quantifier signals were normalized to the quantifier signal of the corresponding internal standard, prior to calculation of concentrations. Only the normalized quantifier signal is an appropriate signal for the calculation of the real concentration. Using the measured standards, a simple regression can be calculated to obtain a calibration curve, and the concentration of the anticipated isotopically labelled analytes can by calculated using this calibration curve (cf. to mass spectra shown in Figures 4 to 7).

EXAMPLE 2 - *Determination of the histamine metabolites using SPE preconcentration*

The sample was treated as described in example 1. 100 µl sample was transferred to new reaction vial, mixed with 300 µl ice-cold MeOH, and centrifuged at minimum 9000 g at 4°C for 10 minutes. The supernatant was transferred to a new vial and mixed with SPE loading buffer, containing 25 mM TRIS acetate buffer pH 9,5 in a ratio of 1:3. The mixed-mode-strong anion exchange SPE cartridge was conditioned with 1 ml MeOH, followed by an equilibration using 1 mL of loading buffer. The sample was loaded onto the equilibrated SPE cartridge, washed with 1 mL loading buffer, 1 mL aqua bidest and 1 mL methanol. The sample was eluted with 2x 250 µl methanol containing 5% formic acid, dried in a rotary evaporator under nitrogen and reconstituted in 25 µL reconstitution solution, comprising 1:3 water/acetonitrile. 25 µl activation mix was prepared as described, added to the sample, and 50 µL derivatization agent (400mM 2-hydrazinoquinoline dissolved in acetonitrile) added. The sample was sample further processed as described in example 1 and the derivatized metabolites determined and quantified.

EXAMPLE 3 *Histamine provocation and measuring total histamine degradation*

The subject is provoked using an oral provocation solution comprising isotopic labeled histamine at a concentration of 0,001 - 0,5 mg/kg, preferably between 0,01 mg/kg and 0,1 mg/kg. Thereafter, urine and serum sample are collected at regular intervals, preferably for 1 - 4h. The samples are then processed and analysed as stated in example 1.

SYNOPSIS

A method of differential diagnosis of histamine intolerance syndrome in a human subject suspected of suffering from histamine intolerance syndrome compared to food allergy, food hypersensitivity or food intolerance. The patient is first subjected to an oral load comprising a defined amount of histamine and stable-isotope labelled histamine. The oral histamine load is preferably typical or equivalent to that of a histamine containing food or beverage. The as well as the diamine/histamine conversion ratio and a diamine/histamine conversion rate per unit time method comprises the steps of (i) obtaining a sample of bodily fluid from said patient after a defined period of time, (ii) derivatization of imidazole acetic acid and methylimidazole acetic acid using 2-hydrazinoquinoline (2HQ) and (iii) determining the amount of isotope-labeled 2-HQ-imidazole acetic acid and 2HQ-methylimidazole acetic acid, to determine the degradation rate of dietary histamine and for differential diagnosis of histamine intolerance syndrome when slow compared to the histamine degradation rate of a healthy individual. The extreme sensitivity of the described method allows a use of a low oral histamine load and monitoring of the histamine degradation via its imidazole metabolites in plasma or serum. The described method allows a measurement of the combined activities of tissue-associated and secreted serum DAO for differential diagnosis of histamine intolerance.

Alternatively, histamine intolerance syndrome can also be diagnosed fully as well as the diamine/histamine conversion ratio and a diamine/histamine conversion rate per unit time. This method comprises the steps of:-

adding to plasma or serum a predefined amount of isotope-labeled DAO substrate, e.g. $^{13}C$-labeled histamine, incubation of said sample at physiological conditions for a predefined period from 1 minute to 10 minutes to obtain oxidative degradation of histamine by the activity of the human DAO enzyme contained in said sample;
, determining by LC-MS/MS technique the concentration of isotope-labeled imidazole acetic acid or methyl imidazole acetic acid and, optionally, other isotope-labeled diamine metabolites, to determine a histamine degradation rate and/or the histamine half-life in serum or plasma..

## Claims

**1.** A method of diagnosis of histamine intolerance syndrome in a subject suspected of suffering from insufficient DAO (diamine oxidase) enzyme activity comprising the steps of:-

administering orally a preparation, solution or suspension containing a known amount of stable isotopic labeled histamine; chemical derivatization of histamine metabolites in a sample of bodily fluid selected from blood, serum, plasma, urine, or saliva that has been obtained after a predefined period of time, using a derivatization agent which reacts with imidazole acetic acid and N-methyl-imidazole acetic acid compounds;
measuring derivatized isotopic labeled histamine metabolites contained in said sample of bodily fluid using mass spectrometry which comprises a LC-MS/MS technique; and
calculating the subjects' DAO activity and/or the subjects' histamine degradation capacity on basis of the amount of isotopic labeled imidazole acetic acid and methyl imidazole acetic acid contained in said sample of bodily fluid, wherein said sample has optionally had proteins removed prior to the step of measuring.

**2.** The method of claim 1, wherein the stable isotopic labeled histamine is selected from histamine $^{15}N$-labeled at positions at N1 and/or N3 or a histamine $^{13}C$-labeled at positions C2, C4, C5, C6, or C7 or a histamine deuterium labeled at anyone or more hydrogen position, or a histamine having a combination of stable isotope labels.

**3.** The method of claim 1 or claim 2, further comprising an immunological determination of secreted human diamine oxidase in serum or plasma.

**4.** The method of any claim 1 to 3, wherein the derivatization agent is selected from hydrazinoquinoline, 2-hydrazinoquinoline, substituted hydrazinoquinoline, 7-chloro-4-hydrazinoquinoline, dialkyl-hydrazinoquinoline, 3,8-diethyl-2-hydrazinoquinoline, 6-fluoro-4-hydrazinoquinoline.

5. The method of any claim 1 to 4, wherein the carboxyl group of the imidazole acetic acid or a derivative thereof is first activated prior derivatization.

6. A method of diagnosis of histamine intolerance syndrome in a subject suspected of suffering from insufficient DAO enzyme activity comprising the steps of:-

adding to a sample of plasma or serum that has been obtained from said subject suspected of suffering from histamine intolerance a predefined amount of stable isotope-labeled DAO substrate as defined in claim 2 to produce a defined concentration of isotope-labeled DAO substrate in said sample;
incubation of said sample for a predefined period under physiological conditions to obtain degradation of said isotope-labeled substrate by the activity of DAO enzyme contained in said sample of plasma or serum;
determining by mass spectrometry which comprises a LC-MS/MS technique the concentration of the isotope-labeled metabolites of histamine isotopic labeled imidazole acetic acid and methyl imidazole acetic acid, following derivatization, to determine the activity of DAO and a histamine conversion rate per unit time for diagnosis of histamine intolerance syndrome.

7. The method of claim 6, comprising a spiking of the sample with an isotope-labeled histamine to achieve a spiked-diamine concentration of from 200 to 500 nmol·litre-1 serum.

8. The method of claim 6 or claim 7, further calculating the activity of soluble human DAO enzyme in said sample at given physiological conditions from the amounts of DAO substrates converted per unit of time and determining the histamine degradation capacity or half-life of histamine and/or DAO substrate in said patient,
to diagnose a histamine intolerance when the patient’s diamine oxidase activity is below 3 enzyme units (U) per milliliter serum or plasma or below 10 enzyme units (U) per milliliter as measured by the immunoassay and/or the half-life of carbon-13 histamine in serum is above a threshold found in a sample of a healthy subject.

9. The method of any claim 6 to 8, comprising an immunological determination of soluble human diamine oxidase in serum or plasma and a determination of the enzyme activity of DAO in serum or plasma.

10. A kit for diagnosis of histamine intolerance syndrome as claimed in any claim 1 to 4, comprising:-

a defined histamine oral load in the form of a solution, dispersion or tablet, which contains a defined amount of stable isotope-labeled histamine selected from histamine $^{15}$N-labeled at positions N1 and/or N3 or a histamine $^{13}$C-labeled at positions C2, C4, C5, C6, or C7 or a histamine deuterium labeled at one or more hydrogen positions;
a derivatization agent for derivatization of imidazole acetic acid and N-methyl imidazole acetic acid selected from the group comprising derivatized or substituted hydrazinoquinolines, hydrazinoquinoline, 2-hydrazinoquinoline, 4- hydrazinoquinoline; 7-chloro-4-hydrazinoquinoline, dialkyl-hydrazinoquinoline, 3,8-diethyl-2-hydrazinoquinoline, 6-fluoro-4-hydrazinoquinoline and
suitable buffers and solutions for sample preparation.

11. A kit for diagnosis of histamine intolerance syndrome in a sample of plasma or serum as claimed in any claim 5 to 8, comprising:-

a histamine spiking solution, which contains a defined amount of stable isotope-labeled histamine selected from histamine $^{15}$N-labeled at positions N1 and/or N3 or a histamine $^{13}$C-labeled at positions C2, C4, C5, C6, or C7 or a histamine deuterium labeled at anyone or more hydrogen position, or a histamine having a combination of stable isotope labels;
a derivatization agent for derivatization of imidazole acetic acid and N-methyl imidazole acetic acid selected from the group comprising derivatized or substituted hydrazinoquinolines, hydrazinoquinoline, 2-hydrazinoquinoline, 7-chloro-4-hydrazinoquinoline, dialkyl-hydrazinoquinoline, 3,8-diethyl-2-hydrazinoquinoline, 6-fluoro-4-hydrazinoquinoline; and
suitable buffers and solutions for sample preparation.

12. A kit as claimed in claim 10 or claim 11, further comprising an internal standard solution of imidazole acetic acid and methyl imidazole acetic acid additionally isotopic labeled at least at one more position compared to the expected isotopic labeled metabolites.

**13.** A kit as claimed in any claim 10 to 12, comprising two to six matrix standards with varying concentration of a mixture of the expected isotopic labeled imidazole acetic acid and methyl imidazole acetic acid.

**14.** A kit as claimed in any claim 10 to 13, comprising an enzyme solution of catalase, peroxidase, and/or alcohol dehydrogenase.

## Patentansprüche

**1.** Verfahren zur Diagnose des Histaminintoleranzsyndroms bei einer Person, bei der der Verdacht besteht, dass sie an einer unzureichenden DAO (Diaminoxidase)-Enzymaktivität leidet, umfassend die Schritte:-

die orale Verabreichung eines Präparats, einer Lösung oder einer Suspension, die eine bekannte Menge von mit stabilen Isotopen markiertem Histamin enthält;
chemische Derivatisierung von Histaminmetaboliten in einer Probe einer Körperflüssigkeit, ausgewählt aus Blut, Serum, Plasma, Urin oder Speichel, die nach einer vordefinierten Zeitspanne erhalten wird, unter Verwendung eines Derivatisierungsmittels, das mit Imidazolessigsäure und N-Methylimidazolessigsäure-Verbindungen reagiert;
Messen der derivatisierten isotopenmarkierten Histaminmetaboliten, die in der Probe der Körperflüssigkeit enthalten sind, unter Verwendung von Massenspektrometrie, die eine LC-MS/MS-Technik umfasst; und
Berechnen der DAO-Aktivität der Probanden und/oder der Histaminabbaukapazität der Probanden auf der Basis der Menge an isotopenmarkierter Imidazolessigsäure und Methylimidazolessigsäure, die in der Probe der Körperflüssigkeit enthalten sind, wobei die Probe vor dem Messschritt gegebenenfalls von Proteinen befreit wurde.

**2.** Verfahren nach Anspruch 1, wobei das mit stabilen Isotopen markierte Histamin ausgewählt ist aus Histamin$^{15}$, das an den Positionen N1 und/oder N3 N-markiert ist, oder einem Histamin$^{13}$, das an den Positionen C2, C4, C5, C6 oder C7 C-markiert ist, oder einem Histamin, das an einer oder mehreren Wasserstoffpositionen deuteriummarkiert ist, oder einem Histamin mit einer Kombination stabiler Isotopenmarkierungen.

**3.** Das Verfahren nach Anspruch 1 oder Anspruch 2 umfasst ferner eine immunologische Bestimmung der sezernierten menschlichen Diaminoxidase in Serum oder Plasma.

**4.** Verfahren nach einem der Ansprüche 1 bis 3, wobei das Derivatisierungsmittel ausgewählt ist aus Hydrazinochinolin, 2-Hydrazinochinolin, substituiertem Hydrazinochinolin, 7-Chlor-4-hydrazinochinolin, Dialkylhydrazinochinolin, 3,8-Diethyl-2-hydrazinochinolin, 6-Fluor-4-hydrazinochinolin.

**5.** Verfahren nach einem der Ansprüche 1 bis 4, wobei die Carboxylgruppe der Imidazolessigsäure oder eines Derivats davon vor der Derivatisierung aktiviert wird.

**6.** Verfahren zur Diagnose des Histaminintoleranzsyndroms bei einer Person, bei der der Verdacht besteht, dass sie an einer unzureichenden DAO-Enzymaktivität leidet, umfassend die folgenden Schritte:-

Zugabe einer vordefinierten Menge eines stabilen isotopenmarkierten DAO-Substrats, wie in Anspruch 2 definiert, zu einer Plasma- oder Serumprobe, die von der Person erhalten wurde, bei der der Verdacht besteht, dass sie an Histaminintoleranz leidet, um eine definierte Konzentration an isotopenmarkiertem DAO-Substrat in der Probe zu erzeugen;
Inkubation der Probe für einen vordefinierten Zeitraum unter physiologischen Bedingungen, um den Abbau des isotopenmarkierten Substrats durch die Aktivität des in der Plasma- oder Serumprobe enthaltenen DAO-Enzyms zu erhalten;
Bestimmung der Konzentration von isotopenmarkierten Metaboliten von Histamin, wie isotopenmarkierte Imidazolessigsäure und Methylimidazolessigsäure , durch Massenspektrometrie, die eine LC-MS/MS-Technik umfasst, nach Derivatisierung, um die Aktivität von DAO und eine Histaminumwandlungsrate pro Zeiteinheit zur Diagnose des Histaminintoleranzsyndroms zu bestimmen.

**7.** Verfahren nach Anspruch 6, das eine Aufstockung der Probe mit einem isotopenmarkierten Histamin umfasst, um eine Aufstockungsdiaminkonzentration von 200 bis 500 nmol-Liter-1 Serum zu erreichen.

**8.** Verfahren nach Anspruch 6 oder 7, wobei ferner die Aktivität des löslichen menschlichen DAO-Enzyms in der Probe bei gegebenen physiologischen Bedingungen aus den Mengen der pro Zeiteinheit umgewandelten DAO-Substrate berechnet und die Histaminabbaukapazität oder Halbwertszeit von Histamin und/oder DAO-Substrat in dem Patienten bestimmt wird,
um eine Histaminintoleranz zu diagnostizieren, wenn die Diaminoxidaseaktivität des Patienten unter 3 Enzymeinheiten (U) pro Milliliter Serum oder Plasma oder unter 10 Enzymeinheiten (U) pro Milliliter liegt, wie durch den Immunoassay gemessen, und/oder die Halbwertszeit von Kohlenstoff-13-Histamin im Serum über einem Schwellenwert liegt, der in einer Probe eines gesunden Subjekts gefunden wurde.

**9.** Verfahren nach einem der Ansprüche 6 bis 8, umfassend eine immunologische Bestimmung der löslichen menschlichen Diaminoxidase in Serum oder Plasma und eine Bestimmung der Enzymaktivität von DAO in Serum oder Plasma.

**10.** Kit für die Diagnose des Histaminintoleranzsyndroms nach einem der Ansprüche 1 bis 4, umfassend:-

eine definierte orale Histaminmenge in Form einer Lösung, Dispersion oder Tablette, die eine definierte Menge an stabilem isotopenmarkiertem Histamin enthält, das ausgewählt ist aus Histamin$^{15}$ , das an den Positionen N1 und/oder N3 N-markiert ist, oder einem Histamin$^{13}$ , das an den Positionen C2, C4, C5, C6 oder C7 C-markiert ist, oder einem Histamin, das an einer oder mehreren Wasserstoffpositionen deuteriummarkiert ist;
ein Derivatisierungsmittel zur Derivatisierung von Imidazol-Essigsäure und N-Methyl-Imidazol-Essigsäure, ausgewählt aus der Gruppe, die derivatisierte oder substituierte Hydrazinochinoline, Hydrazinochinolin, 2-Hydrazinochinolin, 4-Hydrazinochinolin, 7-Chlor-4-hydrazinochinolin, Dialkyl-Hydrazinochinolin, 3,8-Diethyl-2-hydrazinochinolin, 6-Fluor-4-hydrazinochinolin und
geeignete Puffer und Lösungen für die Probenvorbereitung.

**11.** Kit zur Diagnose des Histaminintoleranzsyndroms in einer Plasma- oder Serumprobe nach einem der Ansprüche 5 bis 8, umfassend

eine Histamin-Spiking-Lösung, die eine definierte Menge an mit stabilen Isotopen markiertem Histamin enthält, ausgewählt aus Histamin$^{15}$ , das an den Positionen N1 und/oder N3 N-markiert ist, oder einem Histamin$^{13}$ , das an den Positionen C2, C4, C5, C6 oder C7 C-markiert ist, oder einem Histamin, das an einer oder mehreren Wasserstoffpositionen deuteriummarkiert ist, oder einem Histamin, das eine Kombination stabiler Isotopenmarkierungen aufweist;
ein Derivatisierungsmittel zur Derivatisierung von Imidazol-Essigsäure und N-Methyl-Imidazol-Essigsäure, ausgewählt aus der Gruppe, die derivatisierte oder substituierte Hydrazinochinoline, Hydrazinochinolin, 2-Hydrazinochinolin, 7-Chlor-4-hydrazinochinolin, Dialkyl-Hydrazinochinolin, 3,8-Diethyl-2-hydrazinochinolin und 6-Fluor-4-hydrazinochinolin umfasst; und
geeignete Puffer und Lösungen für die Probenvorbereitung.

**12.** Kit nach Anspruch 10 oder 11, das ferner eine interne Standardlösung von Imidazolessigsäure und Methylimidazolessigsäure umfasst, die zusätzlich an mindestens einer weiteren Position im Vergleich zu den erwarteten isotopenmarkierten Metaboliten isotopenmarkiert ist.

**13.** Kit nach einem der Ansprüche 10 bis 12, umfassend zwei bis sechs Matrixstandards mit unterschiedlicher Konzentration eines Gemischs aus der erwarteten isotopenmarkierten Imidazolessigsäure und Methylimidazolessigsäure.

**14.** Kit nach einem der Ansprüche 10 bis 13, umfassend eine Enzymlösung von Katalase, Peroxidase und/oder Alkoholdehydrogenase.

## Revendications

**1.** Méthode de diagnostic du syndrome d’intolérance à l’histamine chez un sujet suspecté de souffrir d’une activité insuffisante de l’enzyme DAO (diamine oxydase) comprenant les étapes suivantes : -.

administration orale d’une préparation, d’une solution ou d’une suspension contenant une quantité connue d’histamine marquée par un isotope stable ;
dérivatisation chimique des métabolites de l’histamine dans un échantillon de fluide corporel choisi parmi le

sang, le sérum, le plasma, l'urine ou la salive, obtenu après une période de temps prédéfinie, à l'aide d'un agent de dérivatisation qui réagit avec l'acide acétique imidazole et les composés de l'acide acétique N-méthyl-imidazole ;
mesurer les métabolites de l'histamine marqués par un isotope dérivé contenus dans ledit échantillon de fluide corporel en utilisant la spectrométrie de masse qui comprend une technique LC-MS/MS ; et
calcul de l'activité DAO des sujets et/ou de leur capacité de dégradation de l'histamine sur la base de la quantité d'acide imidazole acétique et d'acide méthylimidazole acétique marqués isotopiquement contenus dans ledit échantillon de fluide corporel, dans lequel les protéines ont éventuellement été éliminées de l'échantillon avant l'étape de mesure.

**2.** La méthode de la revendication 1, dans laquelle l'histamine marquée par un isotope stable est choisie parmi l'histamine$^{15}$ N marquée aux positions N1 et/ou N3 ou une histamine$^{13}$ C marquée aux positions C2, C4, C5, C6, ou C7 ou une histamine marquée au deutérium à une ou plusieurs positions d'hydrogène, ou une histamine ayant une combinaison de marques d'isotopes stables.

**3.** La méthode de la revendication 1 ou de la revendication 2, comprenant en outre une détermination immunologique de la diamine oxydase humaine sécrétée dans le sérum ou le plasma.

**4.** Méthode selon l'une des revendications 1 à 3, dans laquelle l'agent de dérivatisation est choisi parmi l'hydrazinoquinoline, la 2-hydrazinoquinoline, l'hydrazinoquinoline substituée, la 7-chloro-4-hydrazinoquinoline, la dialkyl-hydrazinoquinoline, la 3,8-diéthyl-2-hydrazinoquinoline, la 6-fluoro-4-hydrazinoquinoline.

**5.** La méthode de l'une des revendications 1 à 4, dans laquelle le groupe carboxyle de l
'acide imidazole acétique ou d'un dérivé de celui-ci est activé avant la dérivatisation.

**6.** Méthode de diagnostic du syndrome d'intolérance à l'histamine chez un sujet suspecté de souffrir d'une activité enzymatique DAO insuffisante, comprenant les étapes suivantes:-

ajouter à un échantillon de plasma ou de sérum obtenu à partir de ce sujet suspecté de souffrir d'intolérance à l'histamine une quantité prédéfinie de substrat DAO marqué par un isotope stable tel que défini dans la revendication 2 pour produire une concentration définie de substrat DAO marqué par un isotope dans cet échantillon ;
incubation dudit échantillon pendant une période prédéfinie dans des conditions physiologiques pour obtenir la dégradation dudit substrat marqué à l'isotope par l'activité de l'enzyme DAO contenue dans ledit échantillon de plasma ou de sérum ;
détermination par spectrométrie de masse, qui comprend une technique LC-MS/MS, de la concentration des métabolites de l'histamine marqués par l'isotope , tels que l'acide acétique imidazole et l'acide acétique méthylimidazole marqués par l'isotope , après dérivatisation, afin de déterminer l'activité de la DAO et un taux de conversion de l'histamine par unité de temps pour le diagnostic du syndrome d'intolérance à l'histamine.

**7.** La méthode de la revendication 6 comprend l'addition à l'échantillon d'une histamine marquée par un isotope afin d'obtenir une concentration de diamine dopée comprise entre 200 et 500 nmol-litre-1 de sérum.

**8.** La méthode de la revendication 6 ou de la revendication 7 consiste en outre à calculer l'activité de l'enzyme DAO humaine soluble dans ledit échantillon dans des conditions physiologiques données à partir des quantités de substrats DAO convertis par unité de temps et à déterminer la capacité de dégradation de l'histamine ou la demi-vie de l'histamine et/ou du substrat DAO chez ledit patient,
diagnostiquer une intolérance à l'histamine lorsque l'activité de la diamine oxydase du patient est inférieure à 3 unités enzymatiques (U) par millilitre de sérum ou de plasma ou inférieure à 10 unités enzymatiques (U) par millilitre, telle que mesurée par le dosage immunologique, et/ou que la demi-vie de l'histamine au carbone 13 dans le sérum est supérieure à un seuil trouvé dans un échantillon d'un sujet en bonne santé.

**9.** La méthode de l'une des revendications 6 à 8, comprenant une détermination immunologique de la diamine oxydase humaine soluble dans le sérum ou le plasma et une détermination de l'activité enzymatique de la DAO dans le sérum ou le plasma.

**10.** Kit pour le diagnostic du syndrome d'intolérance à l'histamine selon l'une quelconque des revendications 1 à 4, comprenant:- une charge orale d' histamine définie sous forme de solution, de dispersion ou de comprimé, qui

contient une quantité définie d'histamine marquée par un isotope stable, choisie parmi l'histamine$^{15}$ N marquée aux positions N1 et/ou N3 ou une histamine$^{13}$ C marquée aux positions C2, C4, C5, C6 ou C7 ou une histamine marquée au deutérium à une ou plusieurs positions d'hydrogène ;
un agent de dérivatisation pour la dérivatisation de l'acide imidazole acétique et de l'acide N-méthyl-imidazole acétique choisi dans le groupe comprenant les hydrazinoquinolines dérivées ou substituées, l'hydrazinoquinoline, la 2-hydrazinoquinoline, la 4-hydrazinoquinoline, la 7-chloro-4-hydrazinoquinoline, la dialkyl-hydrazinoquinoline, la 3,8-diéthyl-2-hydrazinoquinoline, la 6-fluoro-4-hydrazinoquinoline, et tampons et solutions appropriés pour la préparation des échantillons.

**11.** Kit pour le diagnostic du syndrome d'intolérance à l'histamine dans un échantillon de plasma ou de sérum, selon l'une des revendications 5 à 8, comprenant une solution de dopage à l 'histamine, qui contient une quantité définie d'histamine marquée par un isotope stable, choisie parmi l'histamine$^{15}$ N marquée aux positions N1 et/ou N3 ou une histamine$^{13}$ C marquée aux positions C2, C4, C5, C6, ou C7 ou une histamine marquée au deutérium à une ou plusieurs positions d'hydrogène, ou une histamine ayant une combinaison de marques d'isotopes stables ; un agent de dérivatisation pour la dérivatisation de l'acide imidazole acétique et de l'acide N-méthyl-imidazole acétique choisi dans le groupe comprenant les hydrazinoquinolines
dérivées ou substituées, l'hydrazinoquinoline, la 2-hydrazinoquinoline, la 7-chloro-4-hydrazinoquinoline, la dialkyl-hydrazinoquinoline, la 3,8-diéthyl-2-hydrazinoquinoline, la 6-fluoro-4-hydrazinoquinoline ; et des tampons et des solutions appropriés pour la préparation des échantillons.

**12.** Kit selon la revendication 10 ou la revendication 11, comprenant en outre une solution étalon interne d'acide imidazole acétique et d'acide méthylimidazole acétique marquée isotopiquement au moins à une position supplémentaire par rapport aux métabolites marqués isotopiquement.

**13.** Kit selon l'une des revendications 10 à 12, comprenant deux à six étalons de matrice à concentration variable d'un mélange d'acide imidazole acétique marqué par l'isotope attendu et d'acide méthyl-imidazole acétique.

**14.** Kit selon l'une des revendications 10 à 13, comprenant une solution enzymatique de catalase, de peroxydase et/ou d'alcool déshydrogénase.

**Fig. 1**

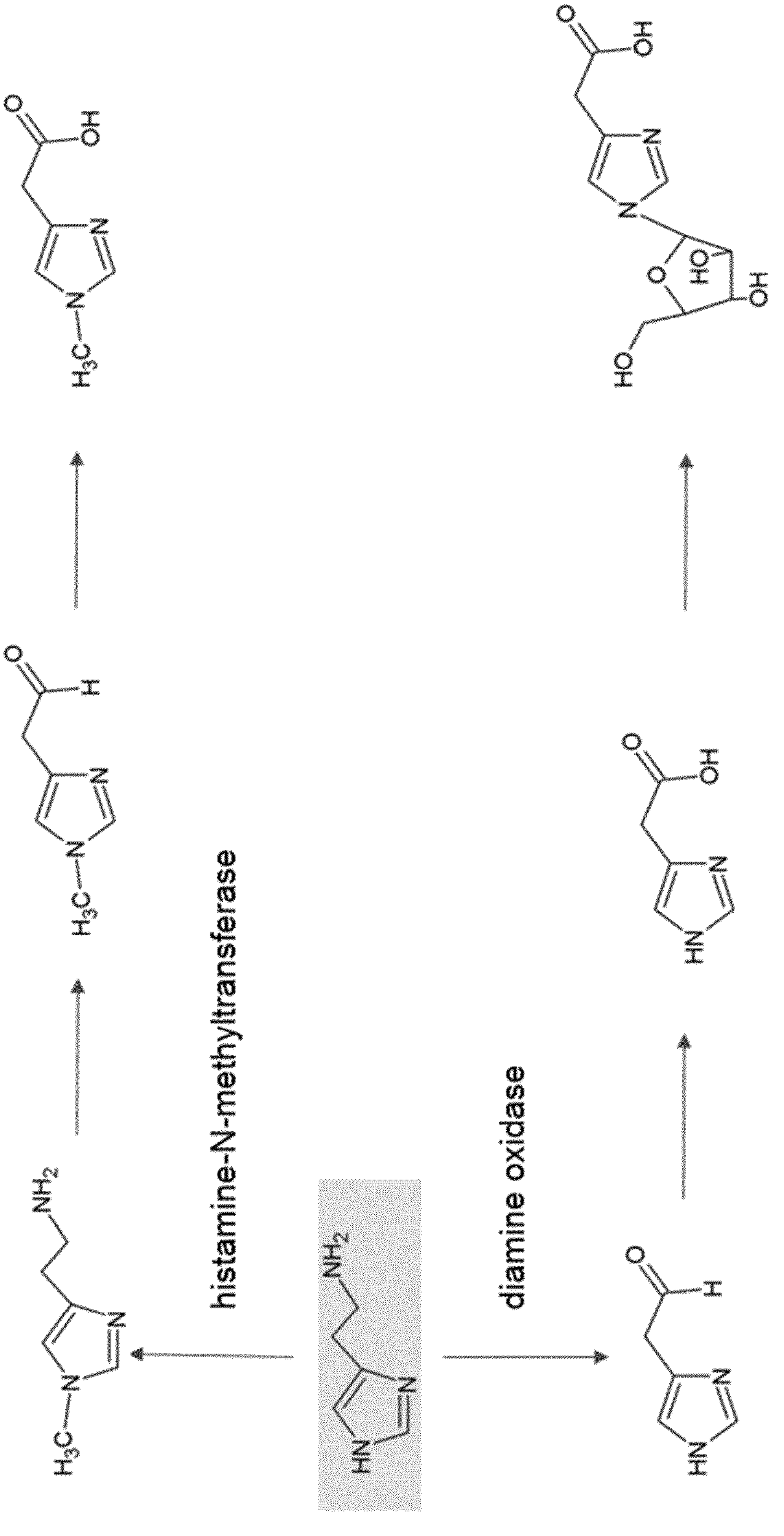

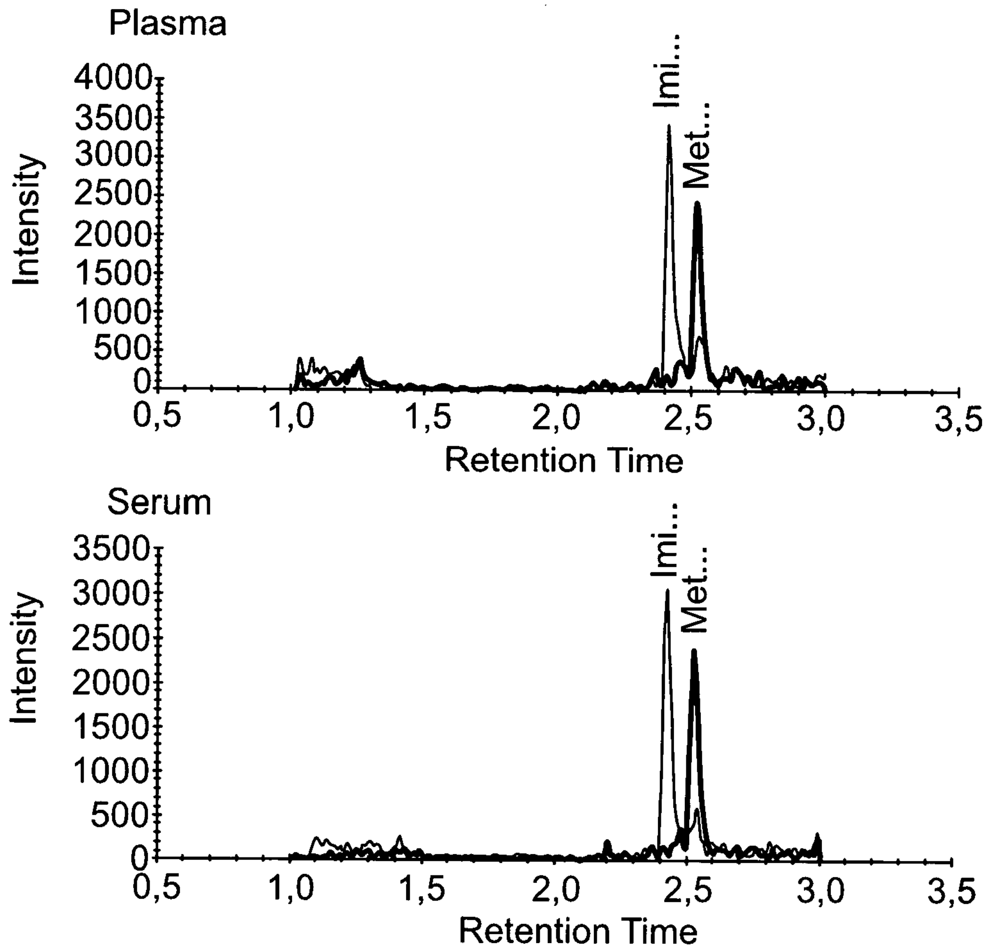
Plasma
Imi...
Met...
Intensity
4000
3500
3000
2500
2000
1500
1000
500
0
0,5
1,0
1,5
2,0
2,5
3,0
3,5
Retention Time
Serum
Imi...
Met...
Intensity
3500
3000
2500
2000
1500
1000
500
0
0,5
1,0
1,5
2,0
2,5
3,0
3,5
Retention Time

Fig. 2

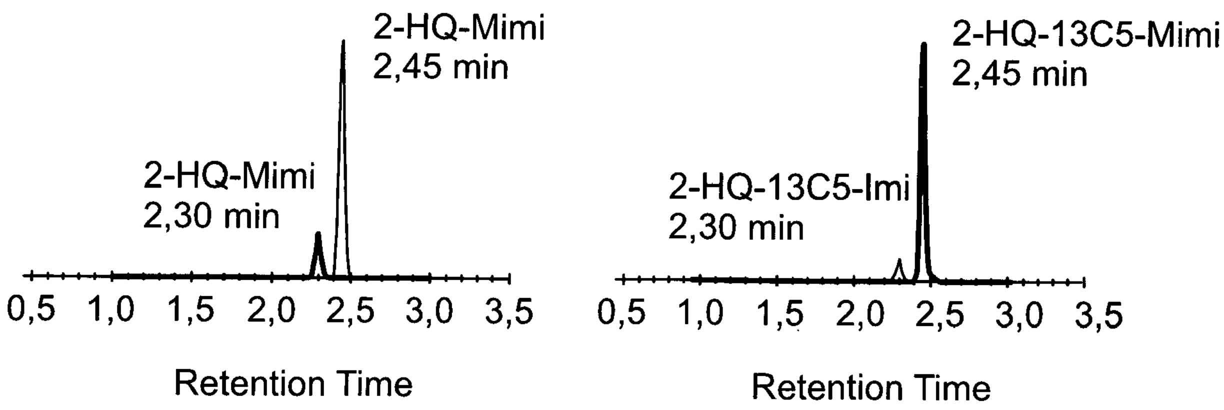
2-HQ-Mimi
2,45 min
2-HQ-Mimi
2,30 min
0,5 1,0 1,5 2,0 2,5 3,0 3,5
Retention Time
2-HQ-13C5-Mimi
2,45 min
2-HQ-13C5-Imi
2,30 min
0,5 1,0 1,5 2,0 2,5 3,0 3,5
Retention Time

Fig. 3

**Fig. 4**

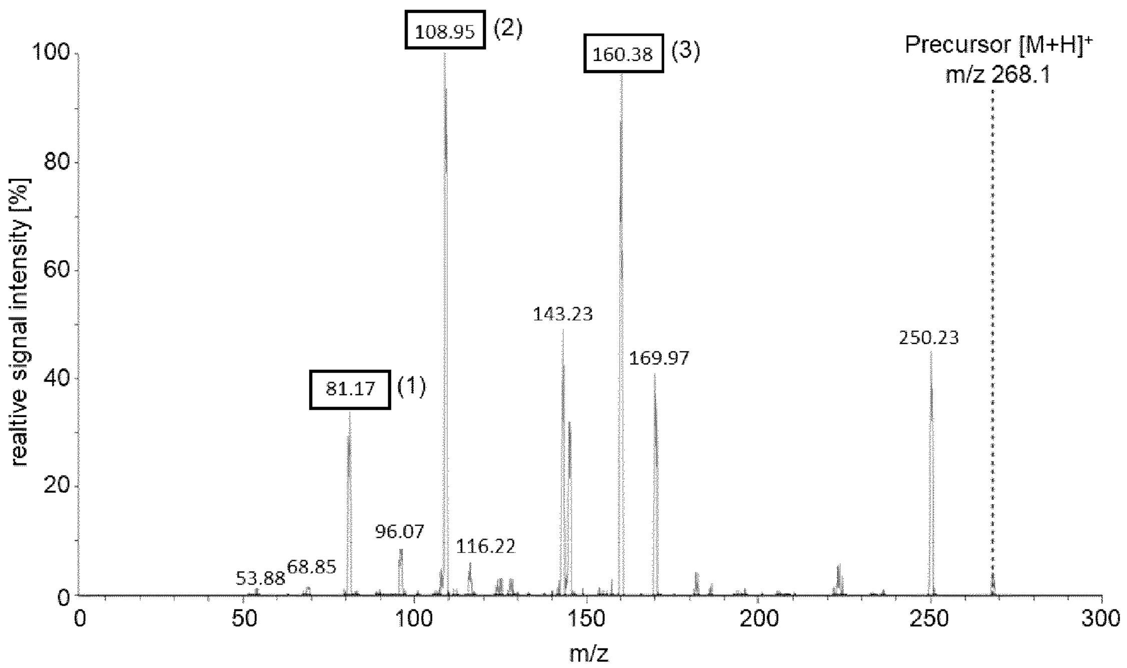
108.95 (2)
160.38 (3)
Precursor [M+H]+
m/z 268.1
81.17 (1)
143.23
169.97
250.23
96.07
116.22
53.88
68.85
realtive signal intensity [%]
m/z

**Fig. 5**

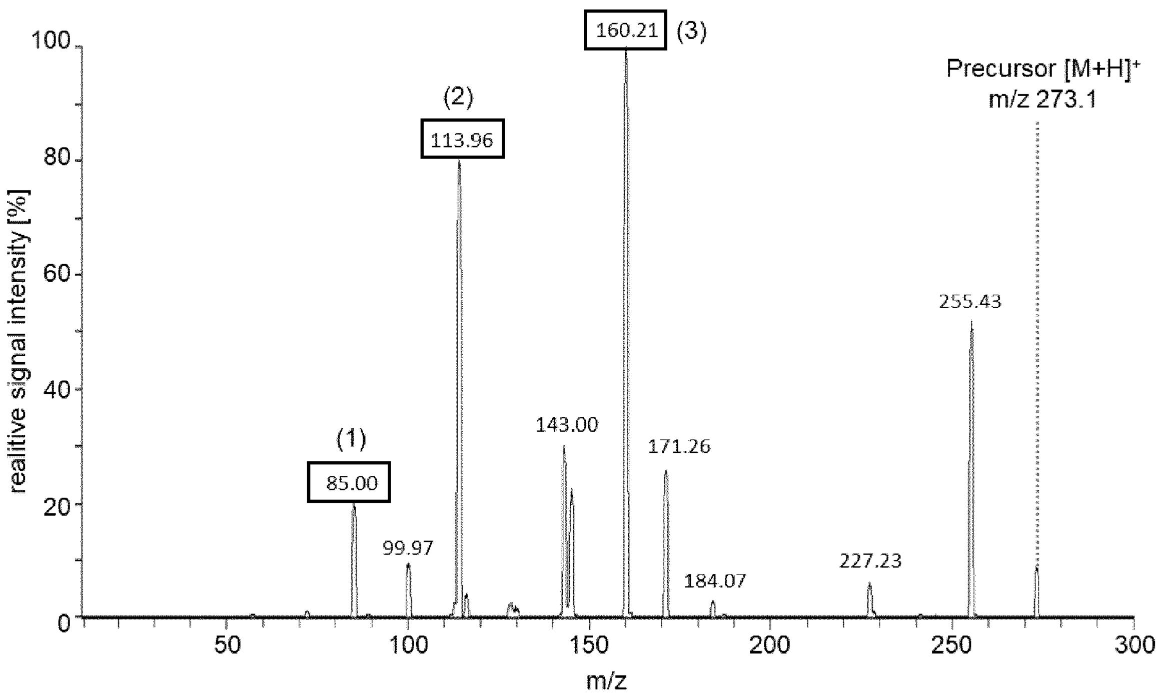
160.21 (3)
Precursor [M+H]+
m/z 273.1
(2)
113.96
255.43
(1)
85.00
143.00
171.26
99.97
184.07
227.23
realtive signal intensity [%]
m/z

# Fig. 6

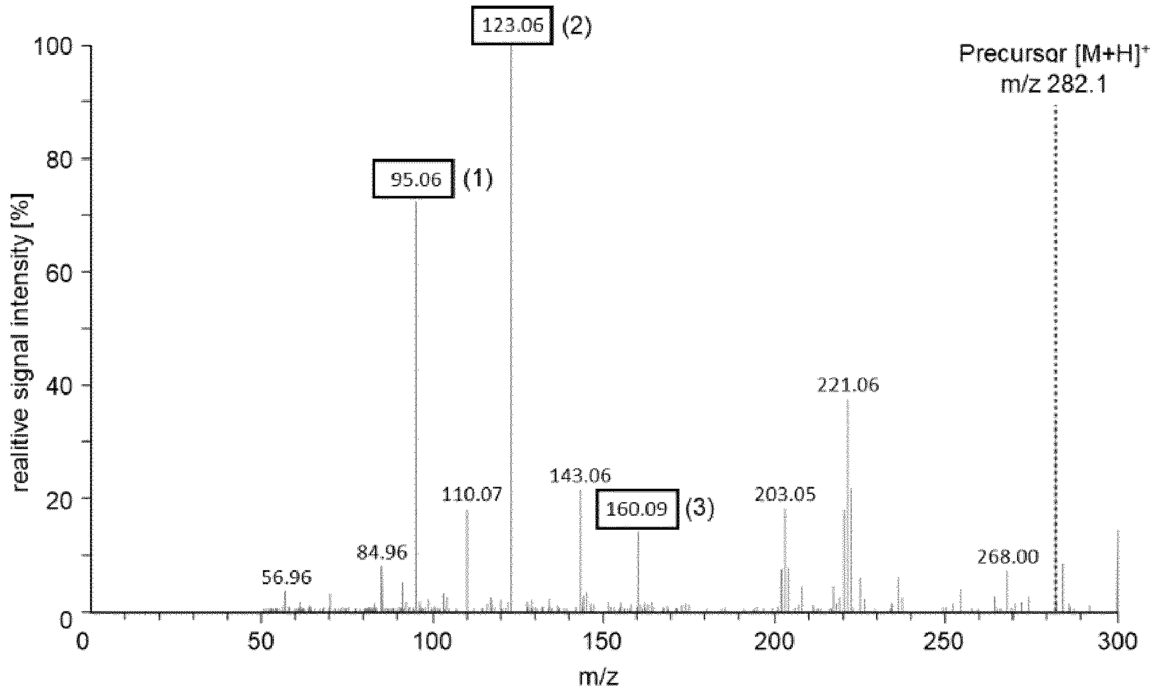
123.06 (2)
Precursor [M+H]+
m/z 282.1
95.06 (1)
221.06
110.07
143.06
160.09 (3)
203.05
84.96
268.00
56.96
realitive signal intensity [%]
100
80
60
40
20
0
0
50
100
150
200
250
300
m/z

# Fig. 7

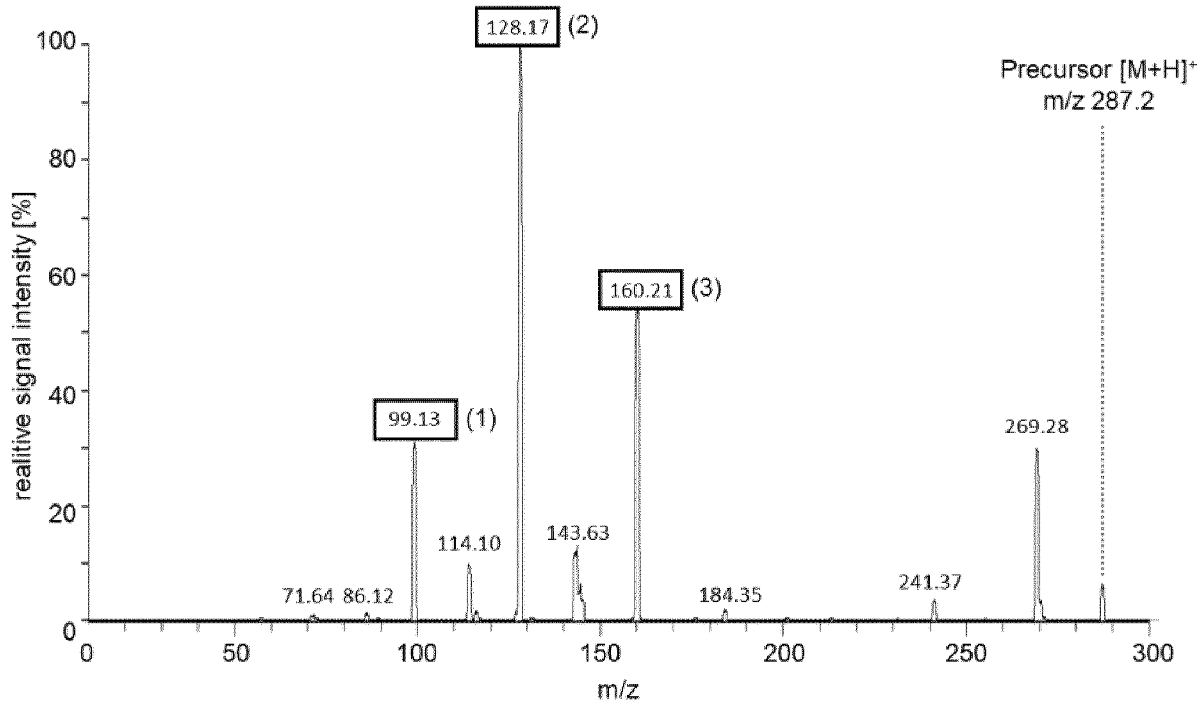
128.17 (2)
Precursor [M+H]+
m/z 287.2
160.21 (3)
99.13 (1)
269.28
114.10
143.63
71.64
86.12
184.35
241.37
realitive signal intensity [%]
100
80
60
40
20
0
0
50
100
150
200
250
300
m/z

Fig. 8

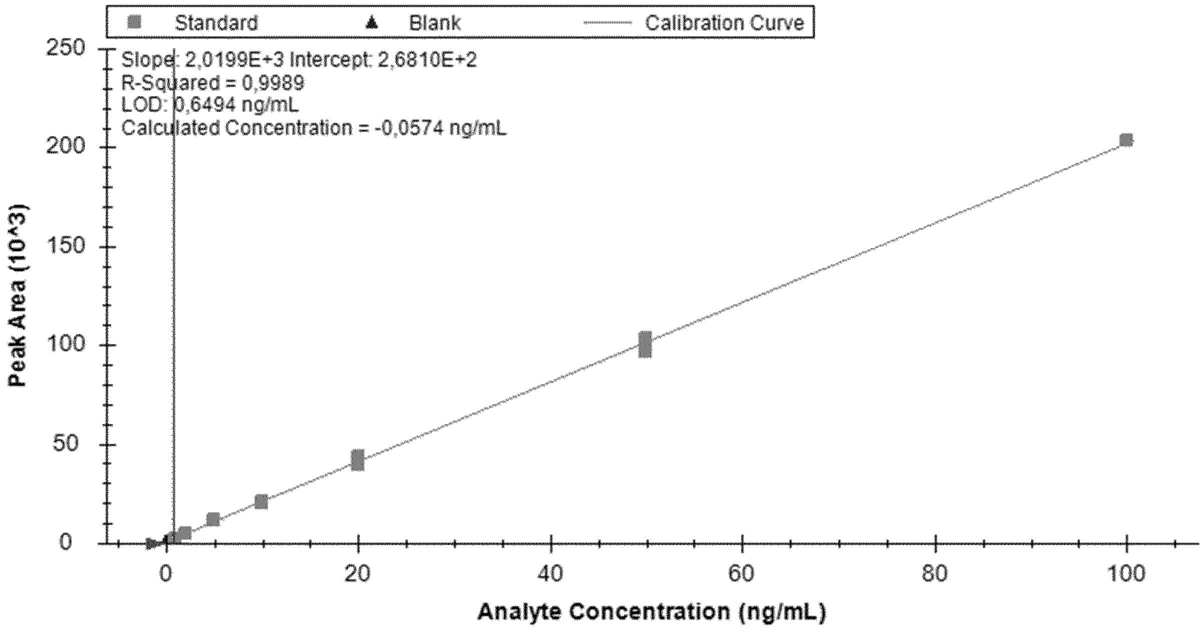
Standard
Blank
Calibration Curve
Slope: 2,0199E+3 Intercept: 2,6810E+2
R-Squared = 0,9989
LOD: 0,6494 ng/mL
Calculated Concentration = -0,0574 ng/mL
Peak Area (10^3)
250
200
150
100
50
0
0
20
40
60
80
100
Analyte Concentration (ng/mL)

Fig. 9

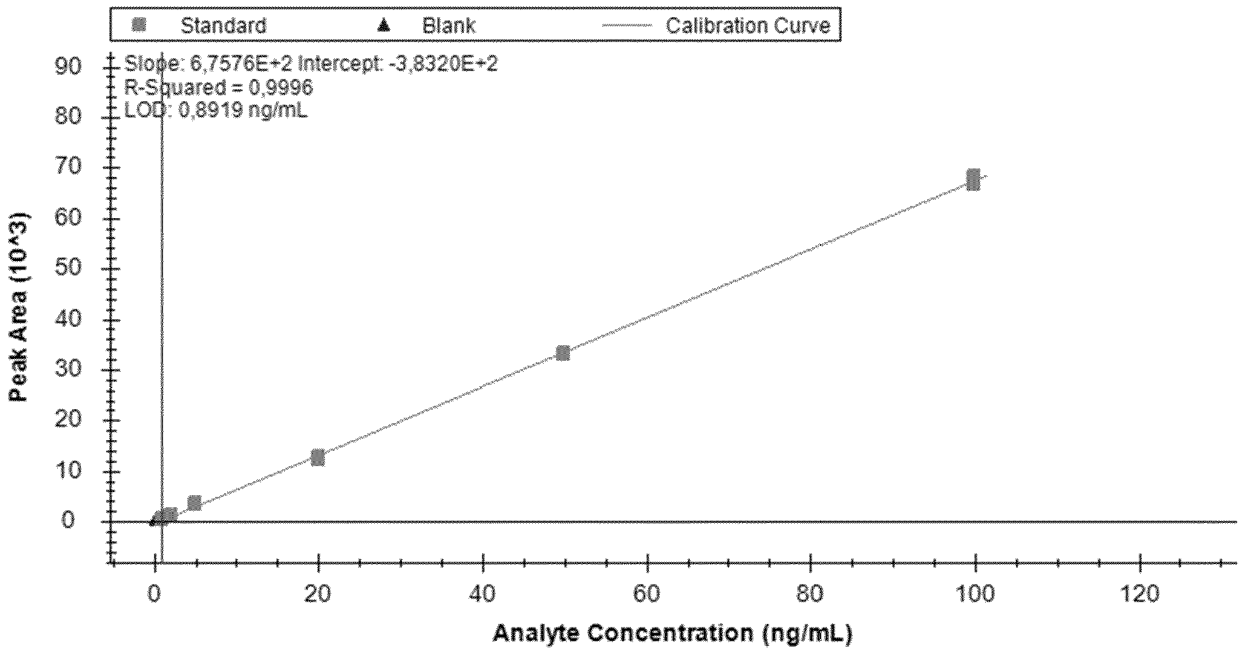
Standard
Blank
Calibration Curve
Slope: 6,7576E+2 Intercept: -3,8320E+2
R-Squared = 0,9996
LOD: 0,8919 ng/mL
Peak Area (10^3)
90
80
70
60
50
40
30
20
10
0
0
20
40
60
80
100
120
Analyte Concentration (ng/mL)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5284749 A, Cowley
- US 4818683 A
- DE 69520383 T2
- RU 2005114081
- WO 2008115044 A
- EP 1948819 A
- WO 2019158718 A
- EP 1948819 B1
- US 2008227116 A1
- US 5124254 A, Hewlins
- US 8003343 B


### Non-patent literature cited in the description


- **SATTLER J et al.** Food induced histaminosis as an epidemiological problem: plasma histamine elevation and hemodynamic alterations after oral histamine administration and blockade of diamine oxidase (DAO). *Agents and Actions,* 1988, vol. 23, 361-65
- **WANTKE F et al.** The red wine maximization test: drinking histamine rich wine induces a transient increase of plasma diamine oxidase activity in healthy volunteers. *Inflamm Res,* 1999, vol. 48, 169-70
- **WANTKE F et al.** The red wine provocation test: intolerance to histamine as a model for food intolerance. *Allergy Proc,* 1994, vol. 15, 27-32
- **WANTKE F et al.** Daily variations of serum diamine oxidase and the influence of H1 and H2 blockers: a critical approach to routine diamine oxidase assessment. *Inflamm Res,* 1998, vol. 47, 396-400
- **JARISCH R et al.** Role of food allergy and food intolerance in recurrent urticaria. *Curr Probl Dermatol,* 1999, vol. 28, 64-73
- **WANTKE F et al.** Histamine free diet: treatment of choice for histamine induced food intolerance and supporting treatment for chronical headaches. *Clin Exp Allergy,* 1993, vol. 23, 982-85
- **GÖTZ M et al.** Histamin-Intoleranz und Diaminooxidasemangel,. *Allergologie,* 1996, vol. 9, 426-30
- **JARISCH R et al.** Histamin-Intoleranz. Histamin und Seekrankheit. Georg Thieme Verl, 2004
- **MCGRATH AP et al.** Structure and Inhibition of Human Diamine Oxidase. *Biochemistry,* 2009, vol. 48, 9810-22
- **ELMORE BO et al.** Human kidney diamine oxidase: heterologous expression, purification, and characterization. *J Biol Inorg Chem,* 2002, vol. 7, 565-679
- **MIZUGUCHI et al.** Purification and characterization of diamine oxidase (histaminase) from rat small intestine. *J. Biochem,* 1994, vol. 116, 631-5
- **KITANAKA et al.** Expression of diamine oxidase (histaminase) in guinea-pig tissues. *Eur J Pharmacol,* 2002, vol. 437, 179-85
- **PINZER TC et al.** Circadian profiling reveals higher histamine plasma levels and lower diamine oxidase serum activities in 24% of patients with suspected histamine intolerance compared to food allergy and controls. *Allergy,* 2018, vol. 73, 949-957
- **ELLMAN GL.** *Arch Biochem Biophys,* 1958, vol. 74, 443-450
- **RIDDLES PW.** *Anal Biochem,* 1979, vol. 94, 75-81
- **MUCKERHEIDE A et al.** *J. Immunol,* 1987, vol. 138, 833
- **APPLE RJ et al.** *J Immunol,* 1987, vol. 140, 290
- **DOMEN PJ.** *J Immunol,* 1987, vol. 139, 195
- **MOREL ; DELAAGE.** Immunoanalysis of histamine through a novel chemical derivatization. *The Journal of Allergy and Clinical Immunology,* 1988, vol. 82, 646-654
- **MOREL et al.** Recognition of imidazole and histamine derivatives by monoclonal antibodies. *Immunol,* 1990, vol. 27, 995-1000
- **HELD, P. et al.** Analysis of Histamine in Wine Samples Using the Microplate Format. *BioTek Instruments,* 28 August 2006
- **KOBAYASHI N et al.** *Adv Clin Chem,* 2001, vol. 36, 139-170
- **CLARET et al.** Homogeneous time resolved fluorescence assay to measure histamine release. *Comb Chem High Throughput Screen,* 2003, vol. 6, 789-94
- **KÜFNER MA et al.** Determination of histamine degradation capacity in extremely small human colon samples. *Inflamm Res,* 2001, vol. 2, 596-7
- **PREVIATI M et al.** Determination of histamine in the whole blood of colon cancer patients. *J Chromatogr,* 2002, vol. 780, 331-9

- **MAYER I et al.** Optimierter Radioextraktionsassay zur quantitativen Bestimmung der Aktivität von Diaminooxidase (DAO) in humanem Serum und Plasma. *Allergologie,* 2005, vol. 28, 1-8
- *Appl Biochem Biotechnol,* November 2007, vol. 143 (2), 164-75
- **HIBI T et al.** Enzymatic assay of histamine by amperometric detection of H202 with a peroxidase-based sensor. *Biosci Biotechnol Biochem,* 2000, vol. 64 (9), 1963-1966
- **KEHOE CA et al.** Plasma diamine oxidase activity is greater in copper-adequate than copper-marginal or copper-deficient rats,. *J. Nutr,* 2000, vol. 130, 30-33
- **KÜFNER MA et al.** Total histamine degradation capacity (THDC) as an important biological marker of histamine metabolism in human colonic mucosa. *Inflamm Res 2002,* vol. 1, 87-81
- **NILSSON B-O et al.** Inhibition of diamine oxidase promotes uptake of putrescine from rat small intestine. *Inflamm Res,* 1996, vol. 45, 513-518
- **NOVOTNY et al.** Diamine oxidase is the amiloride-binding protein and is inhibited by amiloride analogues,. *J Biol Chem,* 1994, vol. 269, 9921-9925
- **OGURI S et al.** Direct detection of endogenous histamine in rat peritoneal mast cells by in-capillary derivatization high-performance capillary electrophoresis. *J Chromatogr B Biomed. Sci Appl,* 1999, vol. 736, 263-71
- **PIETRANGELI P et al.** Inactivation of copper-containing amine oxidases by turnover products. *Eur J Biochem,* 2004, vol. 271, 146-152
- **ZIMATKIN SM et al.** Alcohol-histamine interactions. *Alcohol Alcohol,* 1999, vol. 34 (2), 151-7
- **ELMORE BO et al.** Human kidney diamine oxidase: heterologous expression, purification, and characterization. *J Biol Inorg Chem,* 2002, vol. 7 (6), 565-579
- **HIGASHI T et al.** Simple and practical derivatization procedure for enhanced detection of carboxylic acids in liquid chromatography-electrospray ionization-tandem mass spectrometry. *J Pharm Biomed Anal,* 2010, vol. 52, 809-818
- **CARTWRIGHT AJ et al.** Derivatisation of carboxylic acid groups in pharmaceuticals for enhanced detection using liquid chromatography with electrospray ionisation tandem mass spectrometry. *Rapid Commun Mass Spectrom,* 2005, vol. 19, 1058-1062
- **BARRY SJ et al.** Derivatisation for liquid chromatography-/electrospray mass spectrometry: synthesis of pyridinium compounds and their amine and carboxylic acid derivatives. *Rapid Commun Mass Spectrom,* 2003, vol. 17, 603-620
- **HAYAMA T et al.** Fluorous derivatization combined with liquid chromatography/tandem mass spectrometry: a method for the selective and sensitive determination of sialic acids in biological samples. *Rapid Commun Mass Spectrom,* 2010, vol. 24, 2868-2874